# EUROPEAN PATENT APPLICATION

(11) **EP 4 768 017 A2**
(43) Date of publication of application: **01.07.2026**
(21) Application number: 26165662.3
(22) Date of filing: 25.08.2025
(51) Int. Cl.: A61K 9/00

(54) **NEW CRYSTALLINE FORMS OF A DIPEPTIDYL-PEPTIDASE I INHIBITOR AND ITS USE IN THERAPY**

(30) Priority: 27.08.2024 US 202463687371 P; 26.11.2024 US 202463725067 P; 06.12.2024 EP 24217954; 07.04.2025 EP 25168822
(62) Divisional of application: 25759381.4
(71) Applicant: Boehringer Ingelheim International GmbH, 55216 Ingelheim am Rhein (DE)
(72) Inventor: SAUTER, Wiebke, 55216 Ingelheim am Rhein (DE); CAO, Danting, Ridgefield, 06877-0368 (US); FETSCHER, Alfred, 55216 Ingelheim am Rhein (DE); JIANG, Qi, Ridgefield, 06877-0368 (US); KOEGLER, Harald Peter, 55216 Ingelheim am Rhein (DE); SCHLANGE, Thomas, 55216 Ingelheim am Rhein (DE); SCHMITZ, Jochen, Ridgefield, 06877-0368 (US); SCHNEIDER, Peter, 55216 Ingelheim am Rhein (DE); WETZEL, Kristiane, 55216 Ingelheim am Rhein (DE)
(74) Representative: Lutze, Oliver

(57) **Abstract**

The present invention relates to the dipeptidyl-peptidase I (DPPI or Cathepsin C) inhibitor of formula (I), which is useful in the prevention and/or treatment of bronchiectasis with certain underlying aetiologies, in the prevention and/or treatment of anti-neutrophil cytoplasmic antibody-associated vasculitis or hidradenitis suppurativa using therapeutically efficacious dosages and to polymorphs of Compound (I) as well as to pharmaceutical compositions comprising Compound (I) and its polymorphs.

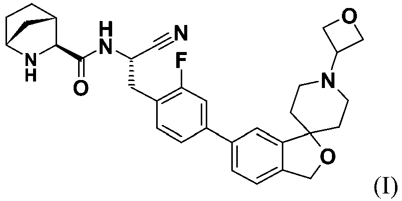

## Description

### FIELD

The present invention relates to the dipeptidyl-peptidase I (DPPI or Cathepsin C) inhibitor of formula (I) (hereinafter also referred to as Compound (I)) in the prevention and/or treatment of bronchiectasis with certain underlying aetiologies, as well as in the prevention and/or treatment of anti-neutrophil cytoplasmic antibody-associated vasculitis and hidradenitis suppurativa using therapeutically efficacious dosages. Moreover, the present invention relates to polymorphs of Compound (I) as well as to pharmaceutical compositions comprising Compound (I) and its polymorphs.

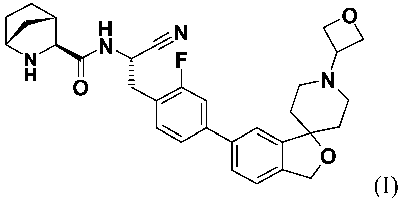

### BACKGROUND

Vintonyak et al WO2016038007A1, which has the International Publication Date of 17th March 2016, discloses DPPI inhibitors (also referred to CatC inhibitors, Cathepsin C inhibitors or dipeptidyl peptidase-1 [DPP-1] inhibitors) including the compound of formula (I) as Example 1 (hereinafter also referred to as Compound (I)), and the preparation thereof, for the treatment of a series of diseases.

Bronchiectasis is a heterogeneous lung disease characterized by abnormal and, in adults, irreversible dilation of the airways. It is characterized by inflammation, impaired mucociliary clearance, and chronic infection. Patients suffer from a high symptom burden (cough, production of large volumes of sputum, dyspnea, chronic fatigue, hemoptysis) and poor quality of life. Recurrent exacerbations of bronchiectasis lead to worsened symptoms, hospitalization, and increased morbidity and mortality. As an effective airway host defense is compromised, *Pseudomonas aeruginosa* can often establish persistent bronchial infection in these patients. *P. aeruginosa* represents one of the most important pathogens across various aetiologies of bronchiectasis, and is associated with high morbidity, frequent exacerbations, and increased mortality rates (Chalmers JD, Goeminne P, Aliberti S, McDonnell MJ, Lonni S, Davidson J, Poppelwell L, Salih W, Pesci A, Dupont LJ, Fardon TC, Soyza A de, Hill AT. The Bronchiectasis Severity Index: an international derivation and validation study. Am J Respir Crit Care Med; 2014; 189(5); 576-585.). The incidence of bronchiectasis is increasing worldwide (Chotirmall SH, Dhar R, McShane PJ, Chang AB. Bronchiectasis: a global disease necessitating global solutions. Lancet Respir Med; 2023; 11(7); 581-583.)

Bronchiectasis covers different aetiologies, including but is not limited to, bronchiectasis patients with underlying genetic disorders [(e.g. primary ciliary dyskinesia (PCD) and cystic fibrosis (CF)], inflammatory diseases (e.g. history of infections) and chronic lung diseases (e.g. asthma and COPD)]. However, the largest single underlying aetiology is not yet determined or idiopathic bronchiectasis. Cystic fibrosis (CF) is a specific aetiology of bronchiectasis, as bronchiectasis is the main manifestation of CF lung disease. Historically, cystic fibrosis bronchiectasis (CFBE) was considered a paediatric disease, and non-cystic fibrosis bronchiectasis (NCFBE) was considered a disease of adults, and consequently bronchiectasis research tended to consider CFBE and NCFBE as separate entities.

Neutrophilic bronchial inflammation is the driver of bronchiectasis progression. Serine proteases released by neutrophils, such as neutrophil elastase (NE), proteinase 3 (PR3), and cathepsin G (CatG), cause structural damage to the airways. These damaged and dilated airways combined with goblet cell metaplasia and mucus hypersecretion and impaired mucus clearance are an ideal growth medium for bacteria due to an ineffective antibacterial defense, leading to chronic infection with pathogens such as *P. aeruginosa*. The end result is a vicious cycle of chronic airway infections, inflammation and progressive airway damage which results in recurrent acute worsening of respiratory symptoms, referred to as acute pulmonary exacerbations (McShane PJ, Naureckas ET, Tino G, Strek ME. Non-cystic fibrosis bronchiectasis. Am J Respir Crit Care Med; 2013; 188(6); 647-656).

The central role of neutrophilic inflammation driven by serine proteases is well described (Jasper AE, McIver WJ, Sapey E, Walton GM. Understanding the role of neutrophils in chronic inflammatory airway disease [version 1; peer review: 2 approved]. F1000 Res; 2019; 8; 557) and the relevance of NE for the pathophysiology of bronchiectasis has been confirmed in a number of studies. NE represents the most abundant elastase in the sputum of chronically inflamed airways in CFBE and NCFBE patients alike and is a key driver of tissue destruction independent of the underlying disease (Hartl D, Latzin P, Hordijk P, Marcos V, Rudolph C, Woischnik M, et al. Cleavage of CXCR1 on neutrophils disables bacterial killing in cystic fibrosis lung disease. Nature Med; 2007; 13(12); 1423-1430).

Sputum NE activity is a biomarker of disease severity and a predictor of risk for exacerbations in patients with bronchiectasis (Diel R, Chalmers JD, Rabe KF, Nienhaus A, Lod-denkemper R, Ringshausen FC. Economic burden of bronchiectasis in Germany. Eur Respir J; 2019; 53; 1802033).

Exacerbations are a hallmark of bronchiectasis, leading to further airway damage and increased mortality. Irreversibly dilated airways, mucus gland hyperplasia, and impaired mucus clearance leads to recurrent acute severe pulmonary infections, referred to as an exacerbation. Even though airway dilation is irreversible in adults, its detrimental consequences might be mitigated, and its progression may be slowed. There is currently no registered therapy that ameliorates neutrophilic inflammation and tissue destruction mediated by uncontrolled neutrophil serine protease (NSP) activity in the airways of patients with bronchiectasis, independent of their underlying aetiology.

The current key pharmacological management option is as-needed antibiotics to treat pulmonary exacerbations. Bronchodilators and/or inhaled corticosteroids are used 'off-label' and dependent on underlying aetiology with limited supportive evidence. Chronic antibiotic treatment, mucoactive therapies, and physiotherapy (airway clearance) are also used. Consequently, the medical need for an efficacious anti-inflammatory treatment for bronchiectasis is high.

NET formation or NETosis is a regulated neutrophil cell death process in which deconden-sated chromatins decorated with lytic granule proteins such as neutrophil serine proteases (NSPs) consisting of Neutrophil Elastase (NE), Cathepsin G (CatG), and Proteinase 3 (PR3) are released into the extracellular space. Notably, two neutrophil granule proteins, NE and myeloperoxidase (MPO), are critical for the initiation of NETosis. In brief, increased intracellular reactive oxygen species (ROS) level upon neutrophil activation leads to liberation of MPO and NE from azurophilic granules into the cytoplasm. These lytic enzymes then move to the nucleus and cleaves histones to facilitate chromatin de-condensation. Intracellular ROS continues to build up as the lytic process develops, which together cause cytoplasmic membrane to rupture and NETs containing NSPs, DNA, and histones released into the extracellular space. Although NETs can trap and facilitate elimination of pathogens, excessive NETosis has pathogenic aspects including immediate cytotoxicity and autoantigenicity (Papayan-nopoulos, V. Neutrophil extracellular traps in immunity and disease. Nat Rev Immunol, 2018, 18, 134-147). NETs are remarkably present in the lesional areas in neutrophilic inflammation-driven diseases such as ANCA-Associated Vasculitis (Kessenbrock, K., Krumbholz, M., Schönermarck, U. et al. Netting neutrophils in autoimmune small-vessel vasculitis. Nat Med, 2009, 15, 623-625) and Hidradenitis Suppurativa (hereinafter HS; Ogawa Y, Muto Y, Kinoshita M, Shimada S, Kawamura T. Neutrophil Extracellular Traps in Skin Diseases. Bio-medicines. 2021; 9(12):1888). In HS, formation of NETs correlates with disease severity (Angel S. Byrd et al. ,Neutrophil extracellular traps, B cells, and type I interferons contribute to immune dysregulation in hidradenitis suppurativa. Sci. Transl. Med, 2019, 11,eaav5908).

Cathepsin C (CatC; also known as Dipeptidyl Peptidase 1), is responsible for the activation of all NSPs during myelopoiesis and neutrophils derived from patients with Papillon-Lefèvre syndrome (PLS) harboring a loss of function CatC mutation are incapable of NETosis (Sorensen O et al; Jerke U et al). Therefore, inhibiting CatC should be a favorable target in diseases driven by neutrophilic inflammation and excessive NETosis-associated damage.

### BRIEF DESCRIPTION OF THE DRAWINGS

**Figure 1****:** TRIAL DESIGN AND SCHEDULE OF VISITS. Figure 1 graphically explains the trial design. "V1" means visit 1, "V2" means visit 2, and so on; "w 0" means week zero, "w 4" means week 4, and so on; "w -6" means week minus six; "V w24" means visit 7, week 24; "V w 36" means visit 8, week 36; "V⁹ w 48" means visit 9, week 48.
**Figure 2****:** DOSE RESPONSE RELATIONSHIP.
   Predicted dose-response curve (on hazard ratio scale) for Compound (I) based on Emax model. The horizontal axis relates to dose in mg. The vertical axis is the placebo-corrected hazard ratio. The solid line is the predicted shape from MCP-Mod. The partially dashed line is the 95% confidence band for the predicted shape from MCP-Mod. The error bars in dotted lines with a black dot indicate the estimates including 95% CIs from Cox regression.

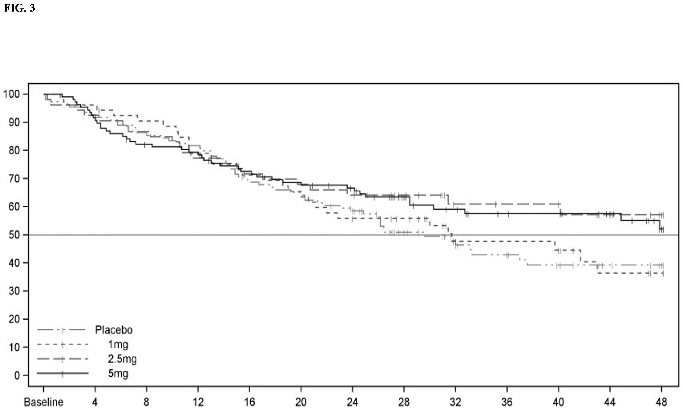

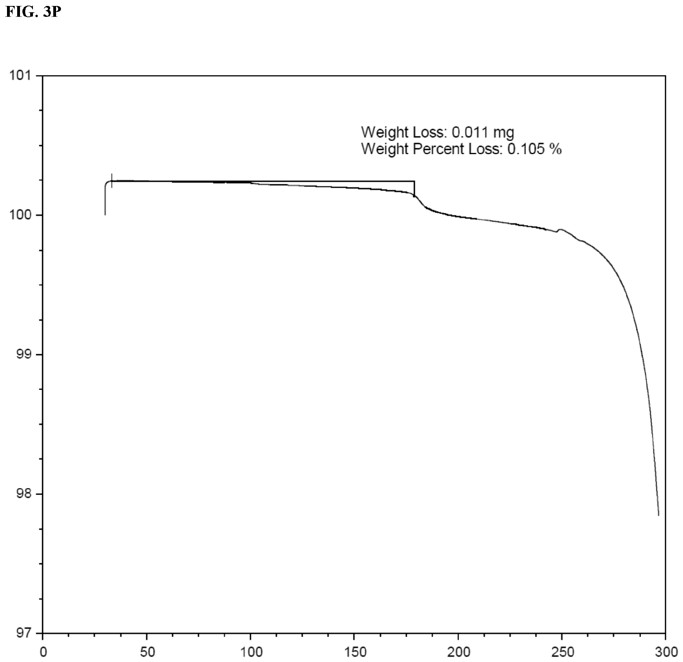

**Figure 3****:** KAPLAN-MEIER PLOT OF TIME TO FIRST PULMONARY EXACERBATION UP TO WEEK 48.
   Estimates were obtained from Cox proportional hazards model adjusted for treatment group, baseline *P. aeruginosa* status and baseline maintenance use of macrolides. All observed data are included in the analysis regardless of intercurrent events. The vertical axis is the proportion of patients without an event. The horizontal axis is the time to first pulmonary exacerbation in weeks. The chart hence maps three different dosages (as indicated in the key) over time compared to placebo.

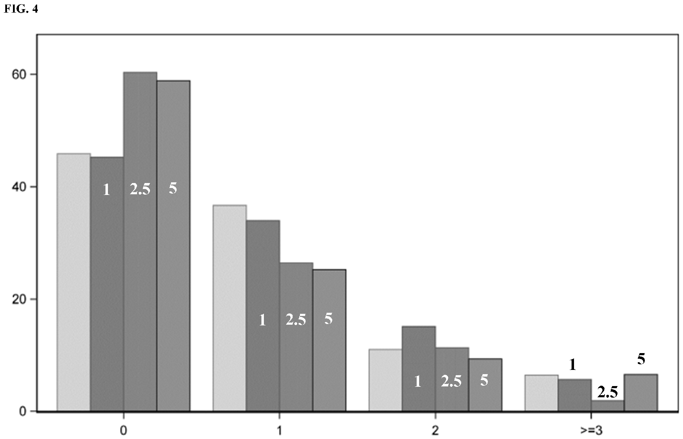

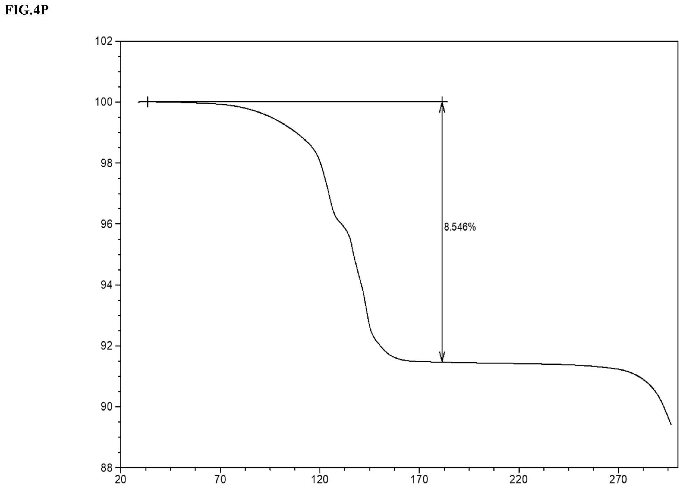

**Figure 4****:** NUMBER OF PULMONARY EXACERBATIONS UP TO WEEK 48.
   Estimates were obtained from a negative binomial regression model adjusted for the categorical variable treatment, baseline *P. aeruginosa* status and baseline maintenance use of macrolides as well as the logarithm of the observational time as an offset. All observed data are included in the analysis regardless of intercurrent events. The vertical axis is the percentage of patients. The horizontal axis is the number of pulmonary exacerbations (PE) up to week 48. The far-lefthand bar for each PE number is coloured in light grey and represents placebo. The three darker bars are the different dosages of Compound (I) namely 1 mg, 2.5 mg and 5 mg, as labelled with 1, 2.5 and 5, respectively.

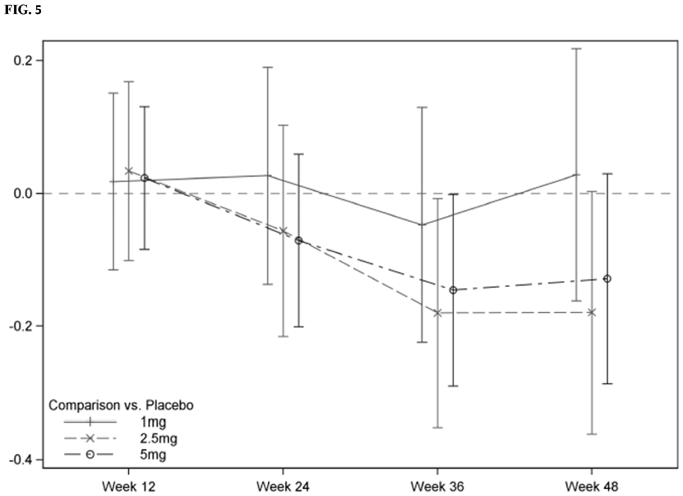

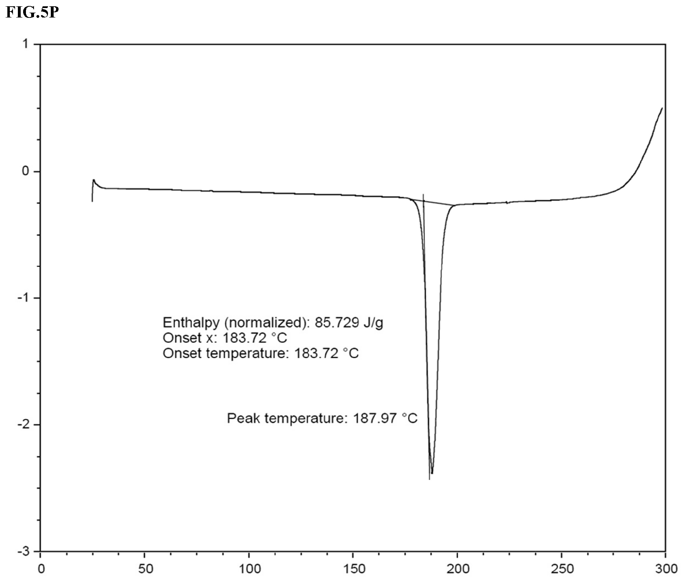

**Figure 5****:** DIFFERENCE IN PROBABILITY OF HAVING AT LEAST ONE PULMONARY EXACERBATION UP TO WEEK 48.
   Estimates were obtained from Kaplan-Meier analysis. The vertical axis is the difference of probability (95% CI) of having at least one pulmonary exacerbation. The horizontal axis is weeks. The three different dosages of Compound (I) namely 1 mg, 2.5 mg and 5 mg are the datapoints as labelled in the key.

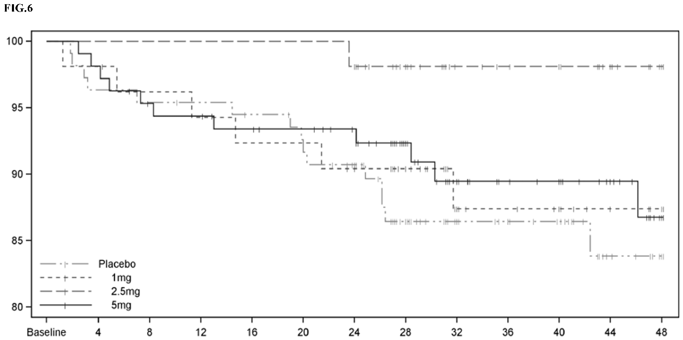

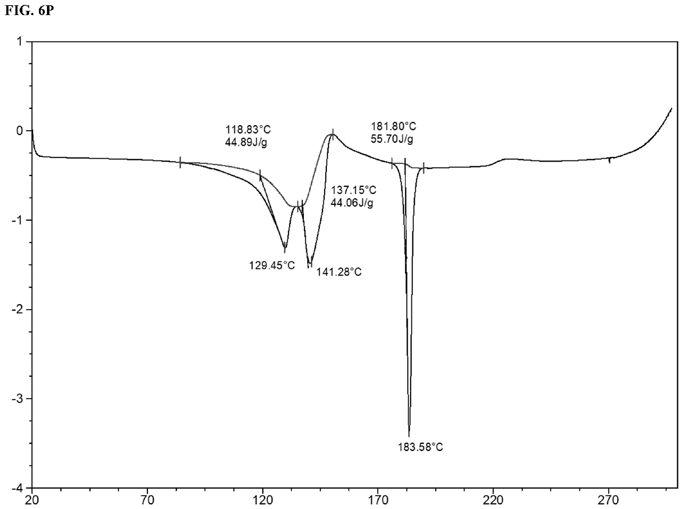

**Figure 6****:** KAPLAN-MEIER PLOT OF TIME TO FIRST SEVERE PULMONARY EXACERBATION UP TO WEEK 48.
   Estimates were obtained from Cox proportional hazards model adjusted for treatment group, baseline *P. aeruginosa* status and baseline maintenance use of macrolides. All observed data are included in the analysis regardless of intercurrent events. The vertical axis is the proportion of patients without event. The horizontal axis is the time to first severe pulmonary exacerbation in weeks. The three different dosages of Compound (I) namely 1 mg, 2.5 mg and 5 mg are charted as labelled in the key compared with placebo.

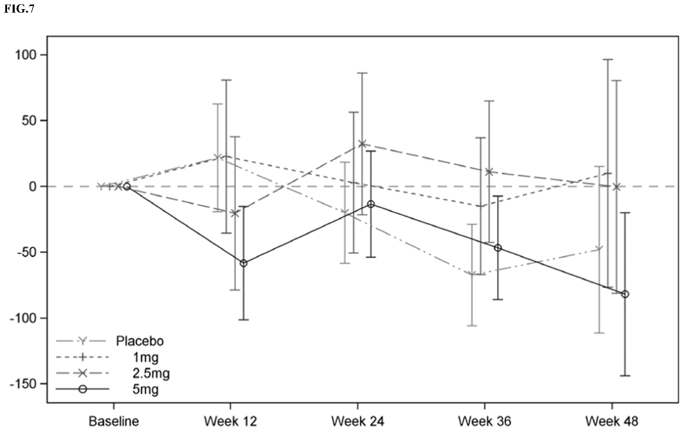

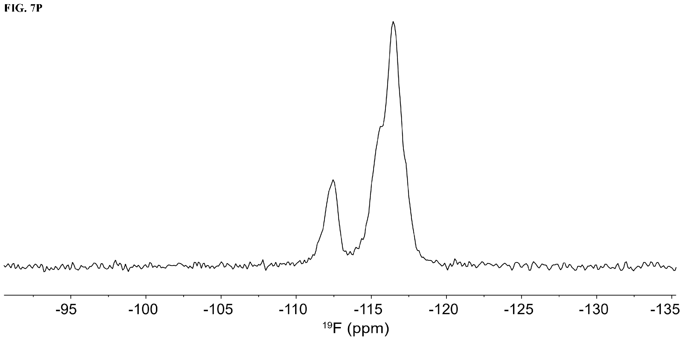

**Figure 7****:** FEV1 in mL over time.
   Estimates were obtained from a mixed model for repeated measures, with *P. aeruginosa* status at baseline and maintenance use of macrolides at baseline as categorical fixed effects, interactions terms for treatment by visit and baseline by visit, and unstructured covariance matrix structure for repeated measurements within participants. The vertical axis is the adjusted mean absolute change from baseline (95% CI) in post-bronchodilator FEV1 (mL). The horizontal axis is weeks. The three different dosages of Compound (I) namely 1 mg, 2.5 mg and 5 mg are the datapoints as labelled in the key, compared with placebo.

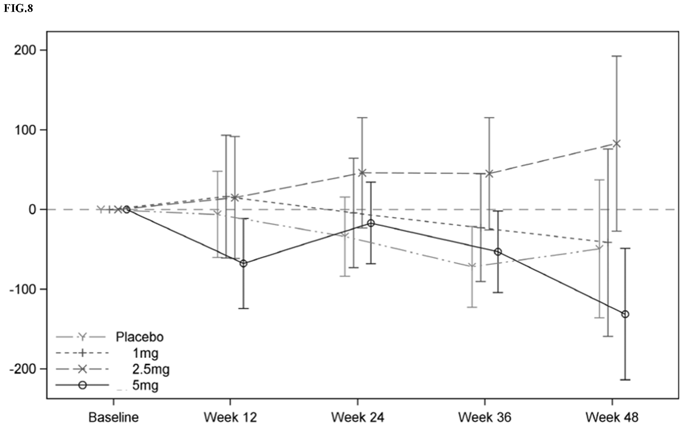

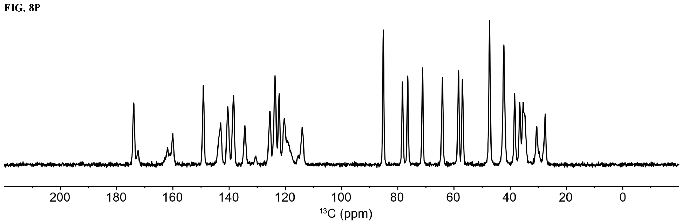

**Figure 8****:** FVC IN ML OVER TIME.
   Estimates were obtained from a mixed model for repeated measures, with *P. aeruginosa* status at baseline and maintenance use of macrolides at baseline as categorical fixed effects, interactions terms for treatment by visit and baseline by visit, and unstructured covariance matrix structure for repeated measurements within participants. The vertical axis is the adjusted mean absolute change from baseline (95% CI) in post-bronchodilator FVC (mL). The horizontal axis is weeks. The three different dosages of Compound (I) namely 1 mg, 2.5 mg and 5 mg are the datapoints as labelled in the key, compared with placebo.

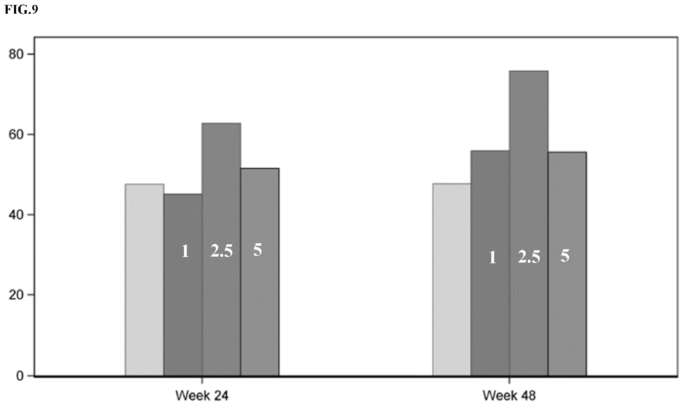

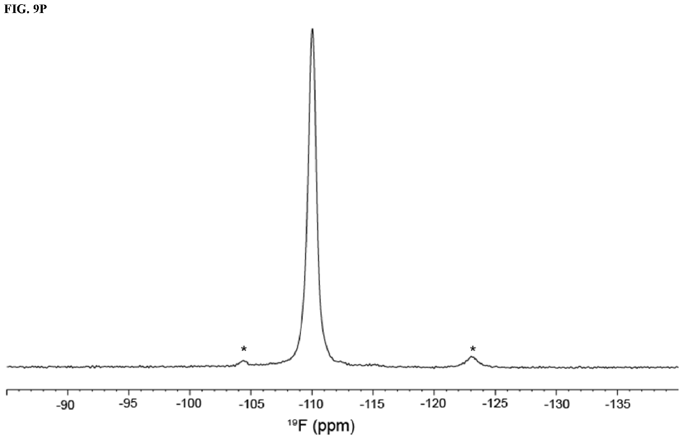

**Figure 9****:** RESPONDER ANALYSES SGRQ TOTAL SCORE AT WEEK 24 AND 48. The SGRQ Symptoms score and SGRQ Total score both range from 0 to 100, where lower scores indicate a better health status. Estimates are obtained from an analysis of covariance model adjusted for baseline SGRQ Symptoms score, treatment, *P. aeruginosa* status at baseline and the maintenance use of macrolides at baseline. A responder was defined as having a ≥4-point decrease from baseline in SGRQ Total score. The vertical axis is the proportion of responders SGRQ Total Score. The horizontal axis is weeks. The far-lefthand bar for each week measured is coloured in light grey and represents placebo. The three darker bars are the different dosages of Compound (I) namely 1 mg, 2.5 mg and 5 mg, as labelled with 1, 2.5 and 5, respectively.

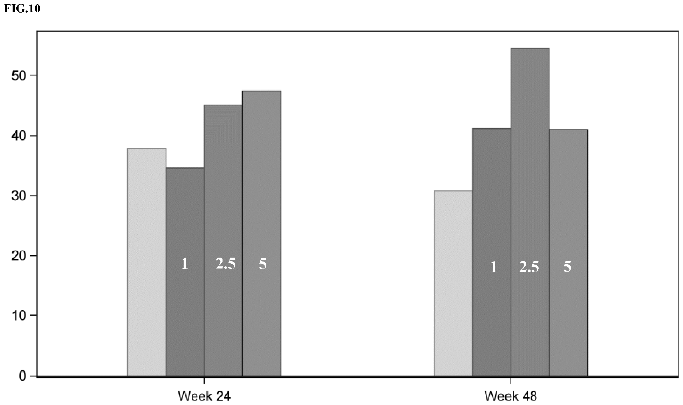

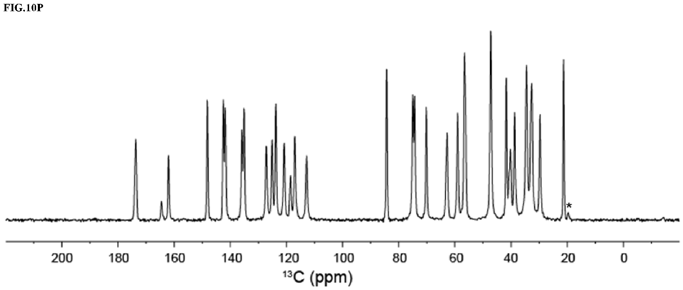

**Figure 10****:** RESPONDER ANALYSES QOL-B RESPIRATORY SYMPTOMS SCORE AT WEEK 24 AND 48.
   The QoL-B respiratory symptoms domain score ranges from 0 to 100, where higher scores indicate a better health status. Estimates were obtained from an analysis of covariance model adjusted for baseline QoL-B respiratory symptoms domain score, treatment, *P. aeruginosa* status at baseline and the maintenance use of macrolides at baseline. A responder was defined as having an ≥8-point change from baseline in QoL-B respiratory symptoms domain score. The vertical axis is the proportion of responders QOL-B Respiratory Symptoms Domain Score. The horizontal axis is weeks. The far-lefthand bar for each week measured is coloured in light grey and represents placebo. The three darker bars are the different dosages of Compound (I) namely 1 mg, 2.5 mg and 5 mg, as labelled with 1, 2.5 and 5, respectively.

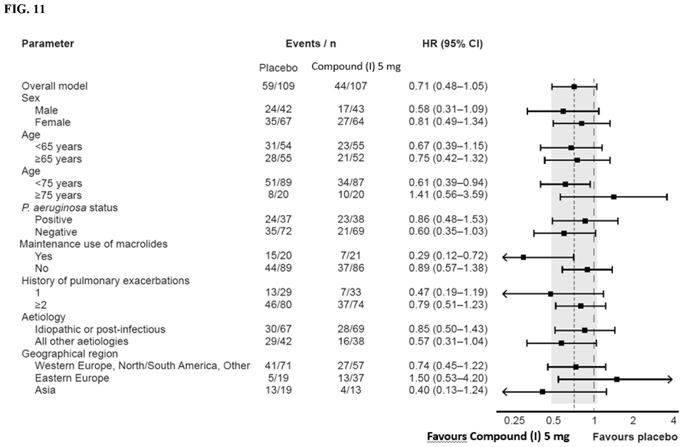

**Figure 11****:** FOREST PLOT OF SUBGROUP ANALYSES FOR THE PRIMARY ENDPOINT (TIME TO FIRST PULMONARY EXACERBATION UP TO WEEK 48) FOR COMPOUND (I) 5 MG VERSUS PLACEBO.
   A HR of less than 1 (indicated by the grey dashed line) favours Compound (I) 5 mg versus placebo. The dotted black line indicates the HR for the overall model. The grey box indicates the 95% CI for the overall model.

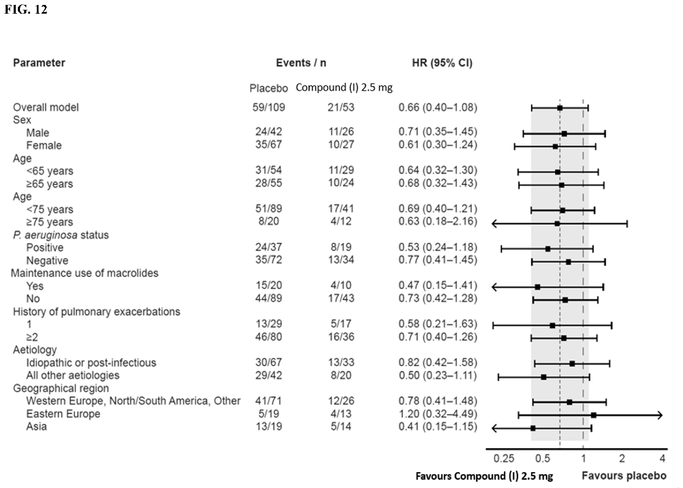

**Figure 12****:** FOREST PLOT OF SUBGROUP ANALYSES FOR THE PRIMARY ENDPOINT (TIME TO FIRST PULMONARY EXACERBATION UP TO WEEK 48) FOR COMPOUND (I) 2.5 MG VERSUS PLACEBO.
   A HR of less than 1 (indicated by the grey dashed line) favours Compound (I) 2.5 mg versus placebo. The dotted black line indicates the HR for the overall model. The grey box indicates the 95% CI for the overall model.

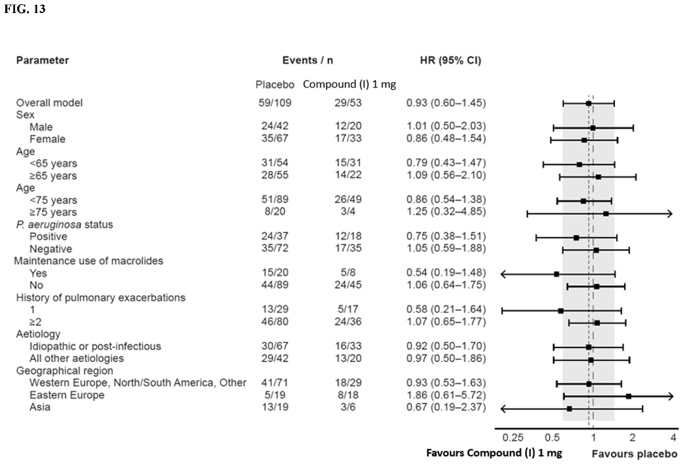

**Figure 13****:** FOREST PLOT OF SUBGROUP ANALYSES FOR THE PRIMARY ENDPOINT (TIME TO FIRST PULMONARY EXACERBATION UP TO WEEK 48) FOR COMPOUND (I) 1 MG VERSUS PLACEBO.
   A hazard ratio (HR) of less than 1 (indicated by the grey dashed line) favours Compound (I) 1 mg versus placebo. The dotted black line indicates the HR for the overall model. The grey box indicates the 95% CI for the overall model.

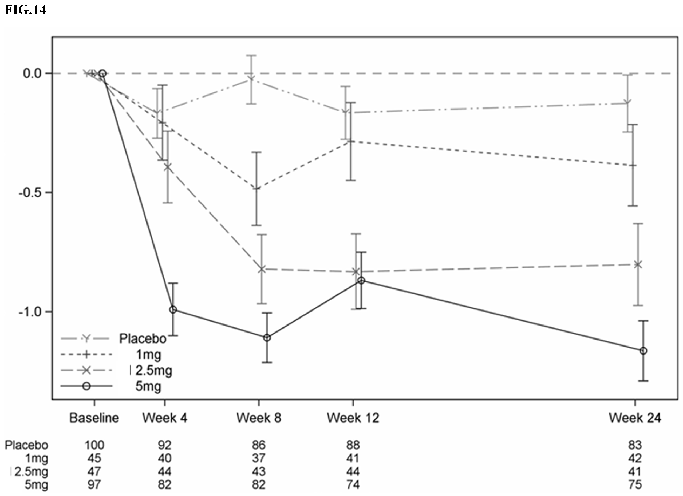

**Figure 14****:** Mixed model with repeated measurements (MMRM) results of change from baseline in log10-transfromed NE activity (relative fluorescent units; RFU) in sputum up to week 48. The vertical axis is the adjusted mean absolute change from baseline (SE) in log 10-transformed NE activity (RFU). Below the chart are the number of patients for each week and at each dosage or placebo.

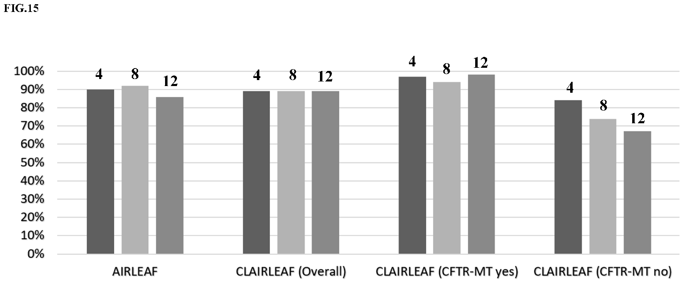

**Figure 15****:** NE Inhibition in sputum up to week 12 in Airleaf and Clairleaf. The bars are in groups of three with the darkest lefthand bar being week 4, the lightest grey middle bar being week 8 and the medium grey bar on the righthand side being week 12.

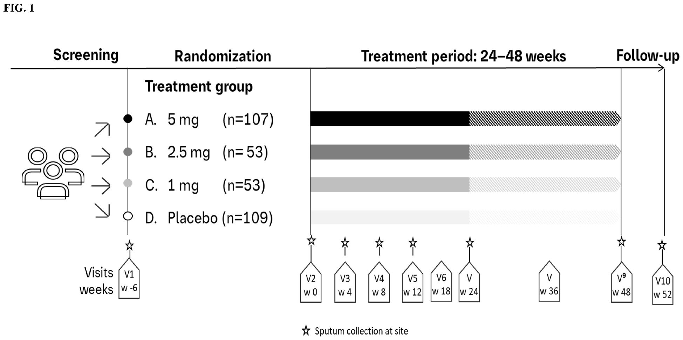

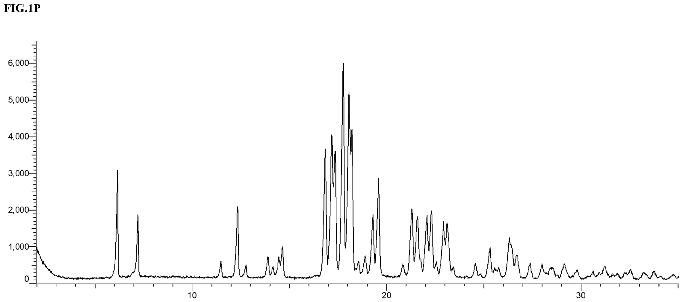

**Figure 1P****:** X-ray powder diffraction diagram (XRPD) of Form [III] (= anhydrous form) of the compound of formula (I). The vertical axis is the counts. The horizontal axis is the 2Theta (Coupled TwoTheta/Theta) WL=1.54060.

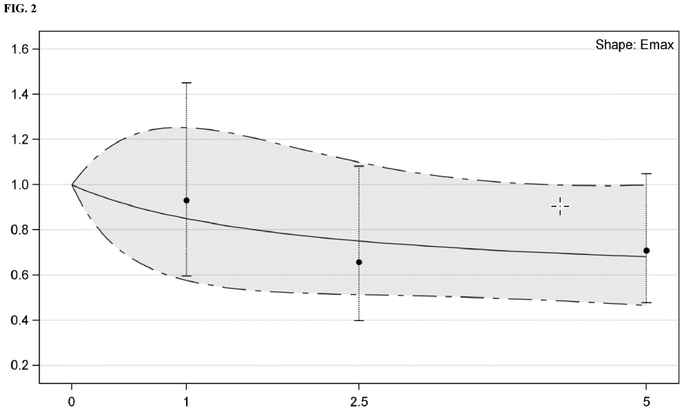

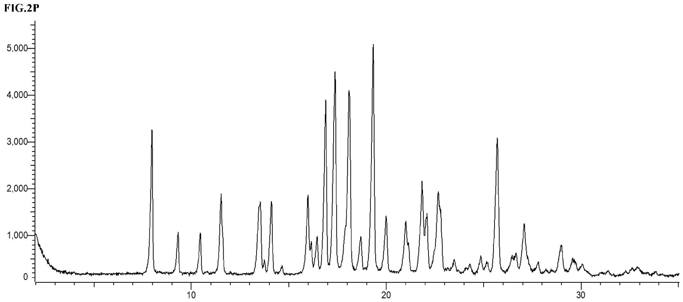

**Figure 2P****:** X-ray powder diffraction diagram (XRPD) of Form [I] (= ethanol solvate) of the compound of formula (I). The vertical axis is the counts. The horizontal axis is the 2Theta (Coupled TwoTheta/Theta) WL=1.54060.
**Figure 3P****:** Thermogravimetric Analysis (TGA) of Form [III] of the compound of formula (I). The vertical axis is weight (%). The horizontal axis is Temperature *T* (°C).
**Figure 4P****:** Thermogravimetric Analysis (TGA) of Form [I] of the compound of formula (I). The vertical axis is weight (%). The horizontal axis is Temperature *T* (°C). Universal V4.5A TA Instruments.
**Figure 5P****:** Differential Scanning Calorimetry (DSC) of Form [III] of the compound of formula (I) (DSC indicates a melt endotherm at about 183.7 °C). The vertical axis is Heat Flow (Normalized) *Q* (W/g)). The horizontal axis is Temperature *T* (°C). Exo Up.
**Figure 6P****:** Differential Scanning Calorimetry (DSC) of Form [I] of the compound of formula (I) (DSC indicates a melt endotherm at about 181.8 °C and two endotherm behaviors at about 119.2 °C and 137.1 °C). The vertical axis is Heat Flow (W/g)). The horizontal axis is Temperature *T* (°C). Universal V4.5A TA Instruments. Exo Up.
**Figure 7P****:** ¹⁹F Solid-State NMR spectrum of Form [III] of the compound of formula (I).
**Figure 8P****:** ¹³C Solid-State NMR spectrum of Form [III] of the compound of formula (I).
**Figure 9P****:** ¹⁹F Solid-State NMR spectrum of Form [I] of the compound of formula (I).
**Figure 10P****:** ¹³C Solid-State NMR spectrum of Form [I] of the compound of formula (I).

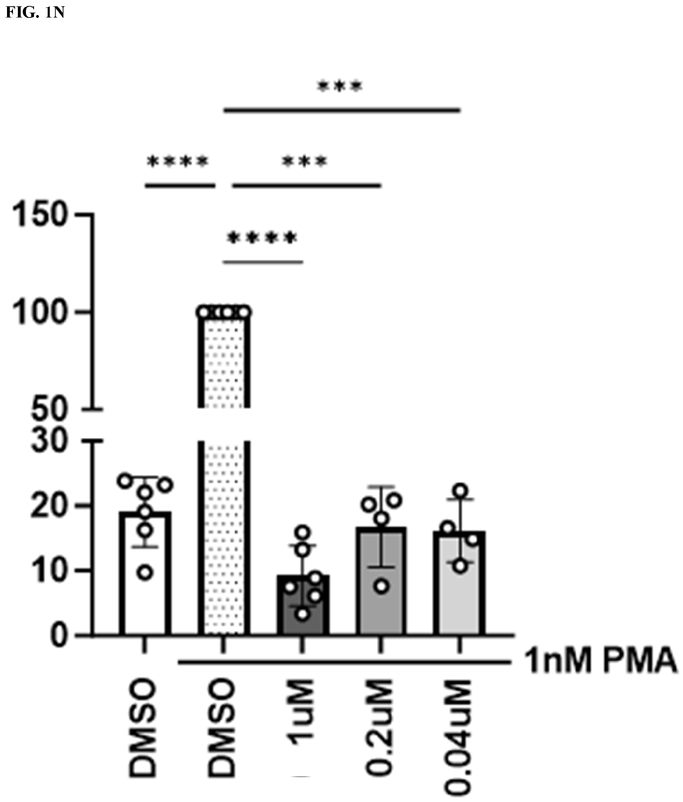

**Figure 1N****:** EFFECT OF COMPOUND (I) ON PMA-INDUCED NETOSIS.
   NETosis was quantified as fluorescence intensity over time for 16 hours following 1nM PMA stimulation. AUC was calculated for each condition (each data point represents an individual donor). The vertical axis of the bar chart indicates the percentage versus AUC of PMA-DMSO. The white bar and the dotted bar are both DMSO only. The three filled bars on the righthand side are compound (I) of three different concentrations namely 1 µM, 0.2 µM and 0.04 µM from left to right. One-way ANOVA was performed normalizing each condition to DMSO-PMA treated condition *p<0.05, **p<0.01, ***p<0.001, ****p<0.0001.

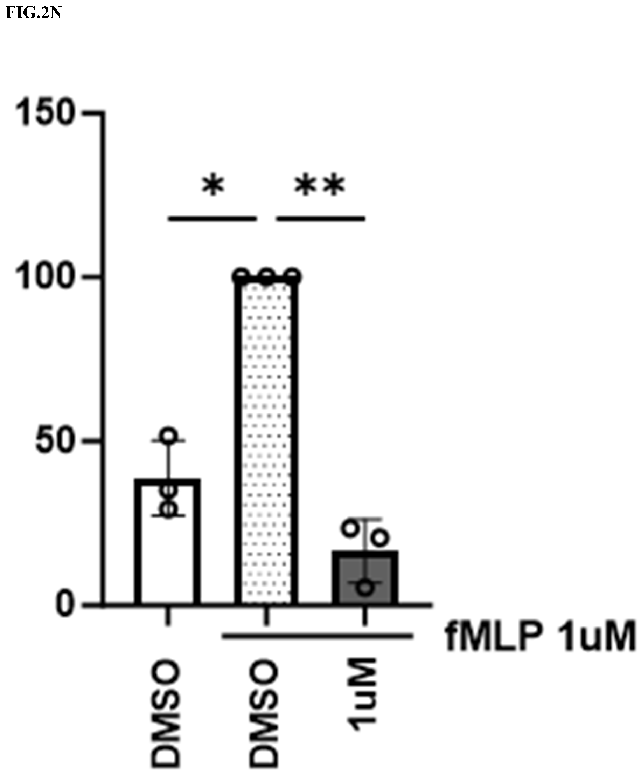

**Figure 2N****:** EFFECT OF COMPOUND (I) ON fMLP-INDUCED NETOSIS.
   NETosis was quantified as fluorescence intensity over time for 22 hours following 1µM fMLP stimulation. AUC was calculated for each condition (each data point represents an individual donor). The vertical axis of the bar chart indicates the percentage versus AUC of fMLP-DMSO. The white bar and the dotted bar are both DMSO only, whereas the filled bar on the righthand side is compound (I) 1µM. One-way ANOVA was performed normalizing each condition to DMSO-fMLP treated condition. *p<0.05, **p<0.01, ***p<0.001, ****p<0.0001.

### DETAILED DESCRIPTION OF THE INVENTION

The objective of the present invention is to provide a dosage for administering the compound of formula (I) 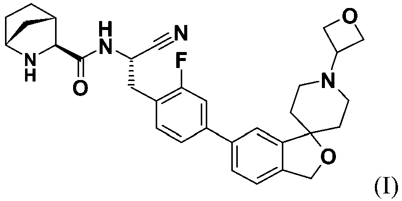 that is therapeutically efficacious in the treatment of bronchiectasis while also showing an acceptable safety and tolerability profile.

In particular the objective of the present invention is to achieve
- a prolongation of the time to first pulmonary exacerbation,
- a reduction in the annual rate of pulmonary exacerbations,
- an improvement of quality of life,
- a beneficial stabilisation of lung function (FEV1 and FVC) and
- a relative change from baseline in NE activity in sputum,
in bronchiectasis patients.

A further objective of the present invention is to provide a compound which may be used in the treatment of HS and ANCA-AV.

A further objective of the present invention is to provide polymorphs of the DPPI-inhibitor of formula (I).

Vintonyak et al WO2016038007A1 is incorporated herein by reference to disclose and describe the methods and/or materials related to Example 1 therein.

Preferably the polymorphs of the DPPI-inhibitor of formula (I) are suitable for large scale production and pharmaceutical formulation.

Preferably the polymorph to be provided shall be stable for extended periods of time without the need for specialized storage conditions.

Vintonyak et al WO2016038007A1 discloses an array of excellent DPPI inhibitors - see, in particular, the IC50 values in Table 9 bridging pages 90 and 91, and the IC50 values in Table 10 bridging pages 94 and 95 of Vintonyak *et al.* Following years of extensive research and development, the inventors surprisingly selected Example 1 from Vintonyak et al WO2016038007A1 as having the best characteristics, properties and efficacy (Example 1 is herein referred to as Compound (I)).

Surprisingly, in a clinical trial investigating efficacy, safety and dosing of the Compound (I) in adults with bronchiectasis, the objective was solved with the 2.5 mg and the 5 mg daily dose.

The 5 mg dose shows a surprisingly good efficacy and a beneficial safety profile.

Especially surprising results are that:
- the 2.5 mg dose shows the best effect on the pulmonary exacerbation risk (Figure 3),
- the 2.5 mg dose shows the best effect on the pulmonary exacerbation rate (Figure 4),
- the 2.5 mg dose shows the best effect on probability to experience a pulmonary exacerbation (Figure 5),
- the 2.5 mg dose shows the best effect on the severe pulmonary exacerbation risk (Figure 6),
- the 2.5 mg dose shows the best effect on duration of antibiotic treatment due to pulmonary exacerbations (Table 8),
- the 2.5 mg dose shows the best effect on the change from baseline in post-bronchodilator ppFEV1 up to week 48 (Table 9),
- the 2.5 mg dose shows the best effect on the change from baseline in post-bronchodilator FEVI1 (mL) up to week 48 (Table 9 and Figure 7),
- the 2.5 mg dose shows the best effect on the change from baseline in post-bronchodilator ppFVC up to week 48 (Table 10),
- the 2.5 mg dose shows the best effect on the change from baseline in post-bronchodilator FVC (mL) up to week 48 (Table 10 and Figure 8),
- the 2.5 mg dose shows the best effect on the change from baseline in SGRQ symptoms and total score at week 24, 36, 48 (Table 11),
- the 2.5 mg dose shows the best effect on SGRQ response based on total score at week 24 and 48 (data for week 24, 36 and 48 in Table 11; Figure 9 at week 24 and 48),
- the 2.5 mg dose shows the best effect on the change from baseline in QoL-B respiratory symptoms score at week 24, 36, 48 (Table 12),
- the 2.5 mg dose shows the best effect QoL-B response based on respiratory symptoms score at week 24 and 48 (data for week 24, 36 and 48 in Table 12; Figure 10 at week 24 and 48), and
- Patients with 1 pulmonary exacerbation and high SGRQ symptoms score in the year before trial treatment benefitted equally good from 2.5 mg and 5 mg compared to patients with 2 or more exacerbations in the previous year (Figure 11 and Figure 12).

The 2.5 mg dose shows the most favourable safety profile compared to the 5 mg daily dose.

Accordingly, the 2.5 mg dose shows consistent better efficacy compared to the 5 mg dose across multiple endpoints while leading to the most favourable safety profile compared to the 5 mg.

Moreover, surprisingly NETosis assay data suggest that inhibition of CatC by Compound (I) can be used for the treatment of HS and ANCA-AV. Based on the bronchiectasis clinical trial described herein the treatment of HS and ANCA-AV is preferably using the 2.5 mg and the 5 mg daily dose.

Moreover, surprisingly novel crystalline forms of the compound of formula (I) (hereinafter also referred to as Compound (I)) have been found.

The major polymorphic form is crystalline Form [III] (hereinafter also referred to as Form [III], alias Form A). Form [III] is the anhydrous form of the free base which was found to have superior properties making it particularly suitable for pharmaceutical development.

A second polymorphic form is crystalline Form [I] (hereinafter also referred to as Form [I], alias Form B), which is the ethanol solvate of Compound (I).

A total of ten polymorphic forms were discovered, but most of these ten polymorphic forms were found to have certain undesirable properties making them less preferred or were not reproducible. Therefore, only Form [III] and the Form [I] are described and characterized herein. Surprisingly, Form [III] was the only anhydrous polymorph isolated. Anhydrous crystalline forms are particularly preferred in oral pharmaceutical compositions. Solvate polymorphs are undesired in oral pharmaceutical compositions.

The present invention thus also relates to crystalline polymorphic forms of Compound (I) 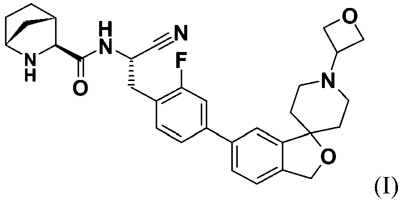 which is
a) an anhydrous form of the free base as Form [III], and
b) an ethanol solvate as Form [I].

The novel crystalline Form [III] has superior dissolution properties and unique solubility characteristics, making it particularly advantageous in large scale production or pharmaceutical formulation processing like the preparation of low drug load tablets or allowing for the manufacture of stable dosage forms containing high levels of drug product*.* This unique solubilization behaviour of Form [III] and the stability of the highly concentrated solutions thereof is a surprising discovery that could not have been predicted beforehand.

Form [III] is also preferred because it provides acceptable physical and chemical solid-state stability under accelerated storage conditions.

The novel crystalline Form [I] represents an ideal crystalline polymorph, which easily crystallizes and can as such be used for purification of Compound (I) material in ethanol solvent. Indeed, crystalline Form [I] is a useful intermediate for purification purposes. Form [I] is also nontoxic and, accordingly, it also has the potential to be used as a pharmaceutical active ingredient.

### USED TERMS AND DEFINITIONS

Terms not specifically defined herein should be given the meanings that would be given to them by one skilled in the art in the light of the disclosure and the context.

All papers, publications and patent rights cited in this specification are herein incorporated by reference and describe the methods and/or materials in the context of the citation.

Pulmonary exacerbations were defined of having three or more of the following symptoms for at least 48 hours resulting in a physician's decision to prescribe antibiotics:
- Increased cough
- Increased sputum volume or change in sputum consistency
- Increased sputum purulence
- Increased breathlessness and/or decreased exercise tolerance
- Fatigue and/or malaise
- Haemoptysis.

Severe pulmonary exacerbations are defined as exacerbations leading to hospitalization and/or intravenous antibiotic administration, or to a fatal outcome.

Patients with only one exacerbation as baseline data are defined as patients with "early disease bronchiectasis".

### List of Abbreviations

- AE: adverse event
- AESI: adverse event of special interest
- ANCA: anti-neutrophil cytoplasmic antibody
- ANCA-AV: ANCA-associated vasculitis
- AUC: area under the curve
- CatC: Cathepsin C
- CFBE: cystic fibrosis bronchiectasis
- CFTR-MT: cystic fibrosis transmembrane conductance regulator (CFTR) modulator treatment
- CI: confidence interval
- CO₂: carbon dioxide
- C_{pre,N}: pre-dose Compound (I) plasma concentration immediately before administration of the N^{th} dose after N-1 doses were administered
- C_{pre,ss},: pre-dose Compound (I) plasma concentration immediately before administration at pharmacokinetic steady state
- °C: degrees Celsius
- CT scan: computed tomography scan
- CV: coefficient of variation
- DMSO: dimethyl sulfoxide
- DTT: dithiothreitol
- ECG: electrocardiogram
- Emax: maximum effect
- Events/n: events number/patient number
- FVC: Forced Vital Capacity
- FEV1: forced expiratory volume in 1 second
- FIG.: figure
- fMLP: N-formyl-Met-Leu-Phe
- G-CSF: granulocyte colony stimulating factor
- GCU: green calibration unit
- HR: hazard ratio
- HS: hidradenitis suppurativa
- MCID: Minimum Clinically Important Difference
- MCP-Mod: multiple comparison procedure with modelling
- MMRM: mixed model with repeated measurements
- µg/mL: microgram per milliliter
- ng/mL: nanogram per milliliter
- mg/mL: milligram per milliliter
- U/mL: unit per milliliter
- mg: milligram
- mL: milliliter
- µL: microliter
- mM: millimolar
- µM: micromolar
- nM: nanomolar
- NCFBE: non cystic fibrosis bronchiectasis
- NE: neutrophil elastase
- n.e.: not estimable
- NET: neturophil extracellular trap
- NSP: neutrophil serine protease
- n/N: number analyzed/total number
- n.s.: not significant
- pbo: placebo
- PBS: phosphate-buffered saline
- PMA: phorbol-12-myristate-13-acetate
- pp: percent predicted
- pt-yrs: patient years
- qd: once daily
- QOL: quality of life
- QOL-B,: Quality of Life Questionnaire-Bronchiectasis
- REP: residual effects period
- RFU: relative fluorescent units
- RR: rate ratio
- SGRQ: St. George's Respiratory Questionnaire
- SSNMR: solid state nuclear magnetic resonance
- TEAE: treatment emergent adverse event
- %: percent

### PREFERRED EMBODIMENTS OF THE INVENTION

A first embodiment relates to a Compound (I),
in any polymorphic form of the free base or mixtures or pharmaceutically acceptable salts or solvates (including hydrates) thereof, 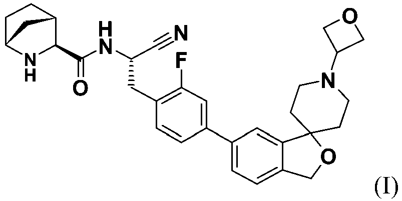
for use in the treatment of a patient suffering from bronchiectasis
wherein compound (I) is administered in a daily dose of 1.5 to 5 mg.

A preferred embodiment relates to Compound (I) for use according to the preceding embodiment wherein Compound (I) is in the form of the free base.

At least one embodiment relates to a compound of formula (I),
or pharmaceutically acceptable salts or solvates thereof, 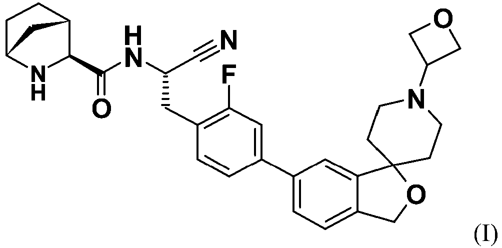
for use in the treatment or prevention of bronchiectasis. In at least one embodiment, the bronchiectasis is selected from the group consisting of non-cystic fibrosis bronchiectasis (NCFBE) and cystic fibrosis bronchiectasis (CFBE).

In at least one embodiment, the treatment or prevention is achieved by administering the compound of formula (I) at a dose of 1.5 mg to 5 mg once per day.

In at least one embodiment, the treatment or prevention is achieved by administering once daily via oral administration one tablet comprising the compound of formula (I).

In at least one embodiment, the one tablet comprises 1.5 mg to 5 mg of the compound of formula (I). In at least one embodiment, the one tablet comprises a total of 2.5 mg of the compound of formula (I). In at least one embodiment, the one tablet comprises a total of 5 mg of the compound of formula (I).

At least one embodiment relates to compound of formula (I),
or pharmaceutically acceptable salts or solvates thereof, 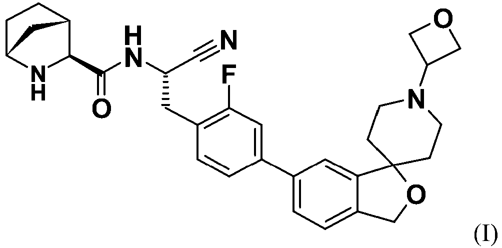
for use in the treatment of a patient suffering from bronchiectasis
wherein compound (I) is administered in a daily dose of 1.5 to 5 mg.

A preferred embodiment relates to Compound (I) for use according to the preceding embodiments, characterized in that Compound (I) is administered in a daily dose of 2.5 mg.

A preferred embodiment relates to Compound (I) for use according to any one of the preceding embodiments, characterized in that Compound (I) is administered in a daily dose of 5 mg.

A preferred embodiment relates to Compound (I) according to any one of the preceding embodiments, for use in the treatment of a patient suffering from non-cystic fibrosis bronchiectasis (NCFBE).

A preferred embodiment relates to Compound (I) according to any one of the preceding embodiments, for use in the treatment of a patient suffering from cystic fibrosis bronchiectasis (CFBE).

Another embodiment relates to an oral pharmaceutical composition consisting essentially of 1 mg, 2.5 mg, or 5 mg of Compound (I), preferably 2.5 or 5 mg, particularly preferred 5 mg, more preferred 2.5 mg of Compound (I),
and optionally of one or more pharmaceutically acceptable carriers or excipients for use in the treatment of a patient suffering from bronchiectasis, wherein this oral pharmaceutical composition is to be administered to the patient once daily.

Another embodiment relates to the use of Compound (I) for the manufacture of an oral pharmaceutical composition consisting essentially of 2.5 mg, or 5 mg of the Compound (I), particularly preferred 5 mg, more preferred 2.5 mg of Compound (I),
and optionally of one or more pharmaceutically acceptable carriers or excipients for the treatment of a patient suffering from NCFBE or CFBE, wherein said oral pharmaceutical composition is to be administered as daily dose.

A preferred embodiment relates to the use of Compound (I) for the manufacture of an oral pharmaceutical composition consisting essentially of 2.5 mg or 5 mg of the Compound (I) and optionally of one or more pharmaceutically acceptable carriers or excipients for the treatment of a patient suffering from NCFBE, wherein said oral pharmaceutical composition is to be administered as daily dose.

A preferred embodiment relates to the use of Compound (I) for the manufacture of an oral pharmaceutical composition consisting essentially of 2.5 mg or 5 mg of the Compound (I) and optionally of one or more pharmaceutically acceptable carriers or excipients for the treatment of a patient suffering from CFBE, wherein said oral pharmaceutical composition is to be administered as daily dose.

A preferred embodiment relates to the use of Compound (I) according to any one of the preceding embodiments, wherein the oral pharmaceutical composition is to be administered to said patient once daily.

A preferred embodiment relates to the use of Compound (I) according to any one of the preceding embodiments, wherein the treatment comprises increasing the time to first pulmonary exacerbation compared to an untreated patient.

A preferred embodiment relates to the use of Compound (I) according to any one of the preceding embodiments, wherein the treatment comprises administration of the 2.5 mg daily dose and increasing the time to first severe pulmonary exacerbation (decreasing the risk) compared to an untreated patient.

A preferred embodiment relates to the use of Compound (I) according to any one of the preceding embodiments, wherein the untreated patient has a history of 1 pulmonary exacerbation at baseline and high symptom score (> 40 SGRQ).

A preferred embodiment relates to the use of Compound (I) according to any one of the preceding embodiments, wherein the untreated patient has a history of 1 pulmonary exacerbation in the previous year and high symptom score (> 40 SGRQ).

A preferred embodiment relates to the use of Compound (I) according to any one of the preceding embodiments, wherein the untreated patient has a history of at least 2 pulmonary exacerbations at baseline.

A preferred embodiment relates to the use of Compound (I) according to any one of the preceding embodiments, wherein the treatment comprises reducing the rate of exacerbations of a patient compared to the rate of exacerbations prior to treatment.

A preferred embodiment relates to the use of Compound (I) according to any one of the preceding embodiments, wherein the rate of pulmonary exacerbations is reduced by at least 10 %.

A preferred embodiment relates to Compound (I) for use according to any one of the preceding embodiments, characterized in that the lung function of the patient is improved compared to the lung function prior treatment.

A preferred embodiment relates to Compound (I) for use according to any one of the preceding embodiments, characterized in that the FEV1 of the patient is stabilized.

A preferred embodiment relates to Compound (I) for use according to any one of the preceding embodiments, characterized in that the FVC of the patient is stabilized.

A preferred embodiment relates to Compound (I) for use according to any one of the preceding embodiments, characterized in that the QOL of the patient assessed by the SGRQ symptoms score (no MCID available) and total score response (MCID of 4 points) is improved compared to the QOL prior treatment.

A preferred embodiment relates to Compound (I) for use according to any one of the preceding embodiments, characterized in that the QOL of the patient assessed by the QoL-B (respiratory symptoms score) response is improved compared to the QOL prior treatment.

A preferred embodiment relates to Compound (I) for use according to any one of the preceding embodiments, characterized in that the antibiotic treatment period due to exacerbations is reduced compared to placebo.

A preferred embodiment relates to Compound (I) for use according to any one of the preceding embodiments, characterized in that the neutrophil elastase activity in sputum of the patient is decreased compared to the activity prior treatment.

A preferred embodiment relates to a quasiquantitative method for the determination of neutrophil elastase (NE) activity in sputum, comprising the steps of:
a) collecting sputum samples from patients;
b) processing the sputum samples using dithiothreitol (DTT) in phosphate buffer;
c) preparing a NE substrate working solution comprising a fluorescent substrate specific to neutrophil elastase activity in TRIS-buffer pH 7.5;
d) preparing a NE stop working solution specific to stop neutrophil elastase activity in TRIS-buffer pH 7.5;
e) combining the sputum samples with the NE substrate working solution to initiate a fluorescence reaction and in parallel with the NE stop working solution for the determination of unspecific background fluorescence;
f) incubating the mixture to allow the enzymatic reaction between neutrophil elastase and the fluorescent substrate under time and temperature adjusted conditions;
g) determining the fluorescence of the samples using a fluorescence detection system; and
h) comparing the total fluorescence with the unspecific background fluorescence.

Another embodiment relates to Compound (I) according to any one of the preceding embodiments for use in the treatment of a patient suffering from early disease bronchiectasis or NCFBE due to one or more diseases selected from the group comprising idiopathic, post-infectious, primary ciliary dyskinesia, primary asthma and primary chronic obstructive pulmonary disease.

Another embodiment relates to Compound (I) according to any one of the preceding embodiments for use in the treatment of a patient suffering from early disease bronchiectasis, and one or more diseases selected from the group comprising primary asthma or primary COPD.

An embodiment relates to Compound (I) for use in the treatment of a patient suffering from hidradenitis suppurativa. Hidradenitis suppurativa is also known as acne inversa.

A preferred embodiment relates to Compound (I) for use in the treatment of a patient suffering from hidradenitis suppurativa, characterized in that Compound (I) is administered in a daily dose of 2.5 mg.

A preferred embodiment relates to Compound (I) for use in the treatment of a patient suffering from hidradenitis suppurativa, characterized in that Compound (I) is administered in a daily dose of 5 mg.

An embodiment relates to Compound (I) for use in the treatment of a patient suffering from ANCA-associated vasculitis.

A preferred embodiment relates to Compound (I) for use in the treatment of a patient suffering from ANCA-associated vasculitis, characterized in that Compound (I) is administered in a daily dose of 2.5 mg.

A preferred embodiment relates to Compound (I) for use in the treatment of a patient suffering from ANCA-associated vasculitis, characterized in that Compound (I) is administered in a daily dose of 5 mg.

An embodiment relates to a crystalline form of the compound of formula (I). An embodiment relates to a crystalline form of the compound of formula (I) for use in the treatment or prevention of a pathology selected from the group consisting of bronchiectasis, NCFBE, CFBE, hidradenitis suppurativa, ANCA-associated vasculitis, idiopathic, post-infectious, or primary ciliary dyskinesia, asthma and chronic obstructive pulmonary disease.

Another embodiment relates to Compound (I) according to any one of the preceding embodiments wherein Compound (I) is present as Form [III], as defined below.

Another embodiment relates to Compound (I) according to any one of the preceding embodiments wherein Compound (I) is present as Form [I], as defined below.

A preferred embodiment relates to the crystalline anhydrous form of Compound (I) as Form [III], having an X-ray powder diffraction pattern comprising peaks at 17.8 ± 0.2 and 18.1 ± 0.2 degrees 2Θ when measured using CuKa radiation.

A preferred embodiment relates to the crystalline anhydrous form of Compound (I) as Form [III], having an X-ray powder diffraction pattern comprising peaks at 17.8 ± 0.2, 18.1 ± 0.2, 12.3 ± 0.2, 7.2 ± 0.2 and 6.2 ± 0.2 degrees 2Θ when measured using CuKa radiation.

A preferred embodiment relates to the crystalline form of Compound (I) as Form [III], having a solid-state NMR spectrum comprising peaks at the following ¹⁹F chemical shifts expressed in parts per million: -112.5 ± 0.2, -116.1 ± 0.2 and -116.5 ± 0.2.

A preferred embodiment relates to the crystalline form of Compound (I) as Form [III], having a solid-state NMR spectrum comprising peaks at the following ¹³C chemical shifts expressed in parts per million: -85.2 ± 0.2, -47.3 ± 0.2 and -42.3 ± 0.2.

A preferred embodiment relates to the crystalline form of Compound (I) as Form [III], having:
both an X-ray powder diffraction pattern comprising peaks at 17.8 ± 0.2, 18.1 ± 0.2, 12.3 ± 0.2, 7.2 ± 0.2, 6.2 ± 0.2, 18.2 ± 0.2, 17.2 ± 0.2,16.8 ± 0.2, 22.1 ± 0.2 and 19.6 ± 0.2 degrees 2Θ when measured using CuKa radiation and
a solid-state NMR spectrum comprising peaks at the following ¹⁹F chemical shifts expressed in parts per million: -112.5 ± 0.2, -116.1 ± 0.2 and -116.5 ± 0.2.

An embodiment relates to a process for preparing the crystalline Compound (I) Form [III], said process comprising crystallization of Compound of formula (I) in n-butyl acetate (nBuOAc).

An embodiment relates to the crystalline Compound (I) Form [III], obtained by or obtainable by the crystallization of Compound of formula (I) in n-butyl acetate (nBuOAc).

An embodiment relates to a pharmaceutical composition comprising the crystalline Compound (I) as Form [III] and at least one pharmaceutically acceptable carrier or diluent,
wherein the crystalline Form [III] has:
(a) an X-ray powder diffraction pattern comprising peaks at 17.8 ± 0.2 and 18.1 ± 0.2 degrees 2Θ when measured using CuKa radiation; or
(b) a solid-state NMR spectrum comprising peaks at the following ¹⁹F chemical shifts expressed in parts per million: -112.5 ± 0.2, -116.1 ± 0.2 and -116.5 ± 0.2, or
(c) both at 17.8 ± 0.2 and 18.1 ± 0.2 degrees 2Θ when measured using CuKa radiation, and a solid-state NMR spectrum comprising peaks at the following ¹⁹F chemical shifts expressed in parts per million: -112.5 ± 0.2, -116.1 ± 0.2 and -116.5 ± 0.2.

At least one embodiment relates to a pharmaceutical composition comprising a tablet having a film coating and a tablet core, wherein the tablet core comprises the compound of formula (I).

In at least one embodiment, the pharmaceutical composition, preferably the tablet core, comprises the crystalline Compound (I) as Form [III].

A preferred embodiment relates to a pharmaceutical composition comprising a film-coated tablet having a tablet core, wherein the tablet core consists of:
(a) 0.5% to 30% (% per tablet) Compound (I) Form [III], in particular a drug load of 1.4% to 3.5%,
(b) 10% to 65% (% per tablet) mannitol, in particular 57% to 63%, preferably the particular types of mannitol 200 or mannitol type fine or mannitol 50,
(c) 10% to 65% (% per tablet) microcrystalline cellulose (MCC), in particular 28% to 33%, preferably particularly the types of microcrystalline cellulose 102, 101, 112, 302, 301 and 110,
(d) 1% to 5% (% per tablet) hydroxypropyl cellulose,
(e) 0.1% to 3% (% per tablet) crospovidone, in particular crospovidone type B, preferably crospovidone in an amount of 0.1% to 0.4%,
   and
(f) 0.5% to 3.5% (% per tablet) magnesium stearate or stearyl fumarate, in particular 0.5% to 1.75% (% per tablet) magnesium stearate or stearyl fumarate.

All percentages are by weight unless specifically indicated otherwise.

An embodiment relates to a tablet core comprising 1.0 wt.% to 4.0 wt.% compound of formula (I) and the remainder to 100 wt.% pharmaceutically acceptable excipients. An embodiment relates to a tablet core comprising 2.0 wt.% to 3.5 wt.% compound of formula (I) and the remainder to 100 wt.% pharmaceutically acceptable excipients. In at least one embodiment, the pharmaceutically acceptable excipients are selected from the group consisting of fillers, binders, disintegrants, lubricants and combinations thereof. In at least one embodiment, the pharmaceutically acceptable excipients are selected from the group consisting of mannitol, microcrystalline cellulose, hydroxypropyl cellulose, crospovidone, magnesium stearate, stearyl fumarate and combinations thereof.

In at least one embodiment, the pharmaceutical composition is a film-coated tablet having a film coating and a tablet core, wherein the tablet core comprises the crystalline form of the compound of formula (I).

An embodiment relates to a process for producing a film-coated tablet comprising:
(a) providing a tablet core, wherein the tablet core comprises the crystalline form of the compound of formula (I);
(b) providing a film coating mixture and a solvent;
(c) preparing a film-coating suspension by suspending the film coating mixture in the solvent;
(d) coating the tablet core in the film-coating suspension;
(e) isolating the film-coated tablet.

In at least one embodiment, the film-coating suspension comprises the film coating mixture and the solvent, wherein the solvent is purified water.

In at least one embodiment, the film coating mixture comprises an agent selected from the group consisting of hydroxypropyl methylcellulose, calcium carbonate, talc, polyethylene glycol, pigment, and combinations thereof. In at least one embodiment, the film coating mixture is substantially free of titaniuim dioxide. In at least one embodiment, the film coating mixture comprises an agent selected from the group consisting of hydroxypropyl methylcellulose, calcium carbonate, talc, polyethylene glycol, pigment, and combinations thereof; and the film coating mixture is substantially free of titaniuim dioxide.

In at least one embodiment, the pharmaceutically acceptable carrier or diluent is selected from the group consisting of mannitol, microcrystalline cellulose, hydroxypropyl cellulose, crospovidone, magnesium stearate, stearyl fumarate, hydroxypropyl methylcellulose, calcium carbonate, talc, polyethylene glycol, pigment and combinations thereof.

An embodiment relates to a process for preparing a film-coated tablet comprising:
(1) providing mannitol, Compound (I) Form [III], hydroxypropylcellulose, crospovidone and microcrystalline cellulose;
(2) blending the ingredients from step (1), preferably in a diffusion mixer;
(3) combining the resulting blend with magnesium stearate to make a final blend;
(4) compressing the final blend into tablet cores, preferably using a rotary tablet press;
(5) coating the tablets in a film-coating suspension.

The person skilled in the art understands that the film-coating suspension applied to the tablet cores will change in its composition as the film coating forms e.g. via drying due to solvent evaporation.

An embodiment relates to a film-coated tablet having a film coating and a tablet core, wherein the tablet core comprises the crystalline form of the compound of formula (I). In at least one embodiment, the film coating comprises an agent selected from the group consisting of hydroxypropyl methylcellulose, calcium carbonate, talc, polyethylene glycol, pigment, and combinations thereof; and the film coating is substantially free of titanium dioxide.

An embodiment relates to a tablet comprising the compound of formula (I) for use in treatment or prevention of a pathology selected from the group consisting of bronchiectasis, NCFBE, CFBE, hidradenitis suppurativa, ANCA-associated vasculitis, idiopathic, post-infectious, primary ciliary dyskinesia, asthma and chronic obstructive pulmonary disease.

An embodiment relates to a pharmaceutical composition comprising the crystalline Compound (I) as Form [III] and at least one pharmaceutically acceptable carrier or diluent, wherein the crystalline Form [III] has:
(a) an X-ray powder diffraction pattern comprising peaks at 17.8 ± 0.2 and 18.1 ± 0.2 degrees 2Θ when measured using CuKa radiation; and
(b) a solid-state NMR spectrum comprising peaks at the following ¹⁹F chemical shifts expressed in parts per million: -112.5 ± 0.2, -116.1 ± 0.2 and -116.5 ± 0.2; and
(c) both at 17.8 ± 0.2 and 18.1 ± 0.2 degrees 2Θ when measured using CuKa radiation and a solid-state NMR spectrum comprising peaks at the following ¹⁹F chemical shifts expressed in parts per million: -112.5 ± 0.2, -116.1 ± 0.2 and -116.5 ± 0.2.

Another embodiment relates to the crystalline ethanol solvate form of Compound (I) as Form [I].

A preferred embodiment relates to the crystalline ethanol solvate form of Compound (I) as Form [I], having an X-ray powder diffraction pattern comprising peaks at 17.4 ± 0.2 and 19.3 ± 0.2 degrees 2Θ when measured using CuKa radiation.

A preferred embodiment relates to the crystalline ethanol solvate form of Compound (I) as Form [I], having an X-ray powder diffraction pattern comprising peaks at 17.4 ± 0.2, 19.3 ± 0.2, 11.5 ± 0.2, 10.5 ± 0.2, 9.3 ± 0.2 and 8.0 ± 0.2 degrees 2Θ when measured using CuKa radiation.

A preferred embodiment relates to the crystalline ethanol solvate form of Compound (I) as Form [I], having a solid-state NMR spectrum comprising a peak at ¹⁹F chemical shift expressed in parts per million: -110 ± 0.2.

A preferred embodiment relates to the crystalline ethanol solvate form of Compound (I) as Form [I], having a solid-state NMR spectrum comprising peaks at the following ¹³C chemical shifts expressed in parts per million: 56.6 ± 0.2, 47.2 ± 0.2 and 21.3 ± 0.2.

An embodiment relates to a process for preparing the crystalline Compound (I) Form [I], said process comprising the following steps:
(a) suspending Compound (I) Form [III] in ethanol over at least 8 hours,
(b) filtrating the suspension,
(c) washing the residue with ethanol and drying,
resulting in the precipitation of Compound (I) Form [I] crystals.

An embodiment relates to a process for preparing the crystalline Compound (I) Form [I], said process comprising the following steps:
(a) suspending Compound (I) Form [III] (1.0 g) in ethanol (5.0 mL) over at least 8 hours,
(b) filtrating the suspension,
(c) washing the residue with ethanol (1.0 mL) and drying,
resulting in the precipitation of Compound (I) Form [I] crystals.

An embodiment relates to a pharmaceutical composition comprising the crystalline Compound (I) as Form [I] and at least one pharmaceutically acceptable carrier or diluent, wherein the crystalline Form [I] has:
(a) an X-ray powder diffraction pattern comprising peaks at 17.4 ± 0.2 and 19.3 ± 0.2 degrees 2Θ when measured using CuKa radiation; or
(b) a solid-state NMR spectrum comprising a peak at ¹⁹F chemical shift expressed in parts per million: -110 ± 0.2; or
(c) both at 17.4 ± 0.2 and 19.3 ± 0.2 degrees 2Θ when measured using CuKa radiation, and a solid-state NMR spectrum comprising a peak at ¹⁹F chemical shift expressed in parts per million: -110 ± 0.2.

An embodiment relates to a pharmaceutical composition comprising the crystalline Compound (I) as Form [I] and at least one pharmaceutically acceptable carrier or diluent, wherein the crystalline Form [I] has:
(a) an X-ray powder diffraction pattern comprising peaks at 17.4 ± 0.2 and 19.3 ± 0.2 degrees 2Θ when measured using CuKa radiation; and
(b) a solid-state NMR spectrum comprising a peak at ¹⁹F chemical shift expressed in parts per million: -110 ± 0.2; and
(c) both at 17.4 ± 0.2 and 19.3 ± 0.2 degrees 2Θ when measured using CuKa radiation, and a solid-state NMR spectrum comprising a peak at ¹⁹F chemical shift expressed in parts per million: -110 ± 0.2.

An embodiment relates to a pharmaceutical composition comprising the crystalline Compound (I) as Form [III] and at least one pharmaceutically acceptable carrier or diluent, wherein the composition is substantially free of the crystalline Compound (I) as Form [I].

"Substantially free from" or "substantially free of' herein means less than 0.5 wt.%, or less than 0.3 wt.%, or about 0 wt.%, by total weight of the composition, formulation, mixture, coating or suspension (*mutatis mutandis*).

Crystalline Compound (I) as Form [I] is particularly useful in the manufacturing process for removing impurities and further optimising the purity of Compound (I) prior to its formulation into a pharmaceutical composition for oral administration in patients. An embodiment relates to the use of crystalline Compound (I) as Form [I] for purifying Compound (I). An embodiment relates to a process comprising:
(a) providing a raw material comprising Compound (I);
(b) suspending the raw material in ethanol for at least 8 hours;
(c) filtering the suspension from step (b);
(d) washing the residue from step (c) with ethanol and subsequently drying the residue;
(e) isolating the crystalline Compound (I) Form [I] as described herein;
(f) taking the crystalline Compound (I) Form [I] and recrystallising in n-butyl acetate (nBuOAc);
(g) isolating the crystalline Compound (I) Form [III] as described herein;
optionally (h) preparing a pharmaceutical composition comprising the crystalline Compound (I) Form [III] from step (g).

The person skilled in the art understands that dose of 5 mg of the compound of formula (I) once daily can also be achieved by administering a tablet comprising 2.5 mg of the compound of formula (I) twice daily. Whilst consumer preference is to consume only one tablet per day, there may be patient-specific reasons why a half dose is preferred twice daily.

The person skilled in the art understands that the pharmaceutical compositions herein disclosed can also be formulated as liquid formulations, such as for patients with swallowing difficulties.

The following clauses are part of the description:
Clause 1. Compound of formula (I), hereinafter Compound (I),
   in any polymorphic form of the free base or mixtures or pharmaceutically acceptable salts or solvates thereof, 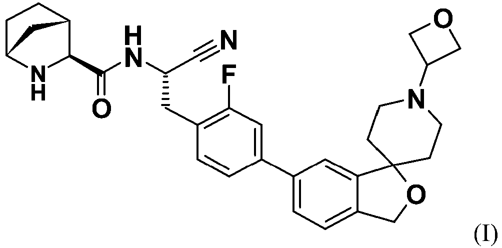
   for use in the treatment of a patient suffering from bronchiectasis
   wherein compound (I) is administered in a daily dose of 1.5 to 5 mg.
Clause 2. Compound (I) for use according to clause 1, wherein Compound (I) is administered in a daily dose of 2.5 mg.
Clause 3. Compound (I) for use according to clause 1, wherein Compound (I) is administered in a daily dose of 5 mg.
Clause 4. Compound (I) according to any of clauses 1 to 3 for use in the treatment of a patient suffering from cystic fibrosis bronchiectasis (CFBE).
Clause 5. Compound (I) according to any of clauses 1 to 3 for use in the treatment of a patient suffering from non-cystic fibrosis bronchiectasis (NCFBE).
Clause 6. An oral pharmaceutical composition consisting essentially of 1 mg, 2.5 mg, or 5 mg of Compound (I)
   and optionally of one or more pharmaceutically acceptable carriers or excipients for use in the treatment of a patient suffering from bronchiectasis, wherein this oral pharmaceutical composition is to be administered to the patient once daily.
Clause 7. Use of Compound (I) for the manufacture of an oral pharmaceutical composition consisting essentially of 2.5 mg or 5 mg of the Compound (I) and optionally of one or more pharmaceutically acceptable carriers or excipients for the treatment of a patient suffering from NCFBE or CFBE, wherein said oral pharmaceutical composition is to be administered as daily dose.
Clause 8. Use of Compound (I) for the manufacture of an oral pharmaceutical composition consisting essentially of 2.5 mg or 5 mg of the Compound (I)
   and optionally of one or more pharmaceutically acceptable carriers or excipients for the treatment of a patient suffering from NCFBE, wherein said oral pharmaceutical composition is to be administered as daily dose.
Clause 9. Use of Compound (I) according to clause 7 or 8, wherein the oral pharmaceutical composition is to be administered to said patient once daily.
Clause 10. Use of Compound (I) according to any of clauses 7 to 9, wherein the treatment comprises increasing the time to first pulmonary exacerbation compared to an untreated patient.
Clause 11. Use of Compound (I) according to any of clause 7 to 10, wherein the untreated patient has a history of 1 pulmonary exacerbation at baseline.
Clause 12. Use of Compound (I) according to any of clause 7 to 10, wherein the untreated patient has a history of at least 2 pulmonary exacerbations at baseline.
Clause 13. Use of Compound (I) according to any of clauses 7 to 12, wherein the treatment comprises reducing the rate of exacerbations of a patient compared to the rate of exacerbations prior to treatment.
Clause 14. Use of a Compound (I) according to Clause 13, wherein the rate of pulmonary exacerbations is reduced by at least 10 %.
Clause 15. Compound (I) according to Clause 1 to 5 for use in the treatment of a patient suffering from early disease bronchiectasis, CFBE or NCFBE due to underlying aetiologies selected from the group comprising idiopathic, post-infectious, primary ciliary dyskinesia; asthma and chronic obstructive pulmonary disease.
Clause 16. Crystalline form of the compound of formula (I) 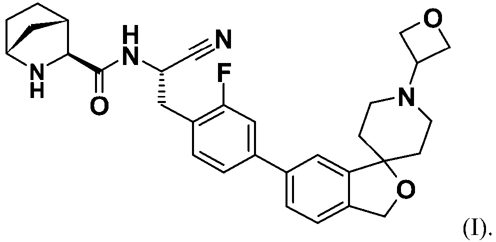
Clause 17. The crystalline anhydrous form of the compound of formula (I) according to clause 16, characterized therein that it is Form [III], having an X-ray powder diffraction pattern comprising peaks at 17.8 ± 0.2 and 18.1 ± 0.2 degrees 2Θ when measured using CuKa radiation.
Clause 18. The crystalline anhydrous form of the compound of formula (I) according to clause 17, characterized therein that it is Form [III], having an X-ray powder diffraction pattern comprising peaks at 17.8 ± 0.2, 18.1 ± 0.2, 12.3 ± 0.2, 7.2 ± 0.2 and 6.2 ± 0.2 degrees 2Θ when measured using CuKa radiation.
Clause 19. The crystalline form of the compound of formula (I) according to clause 16, characterized therein that it is Form [III], having a solid-state NMR spectrum comprising peaks at the following ¹⁹F chemical shifts expressed in parts per million: -112.5 ± 0.2, -116.1 ± 0.2 and -116.5 ± 0.2.
Clause 20. The crystalline form of the compound of formula (I) according to clause 19, characterized therein that it is Form [III], having a solid-state NMR spectrum comprising peaks at the following ¹³C chemical shifts expressed in parts per million: -85.2 ± 0.2, -47.3 ± 0.2 and -42.3 ± 0.2.
Clause 21. The crystalline form of the compound of formula (I) according to clause 16, characterized therein that it is Form [III], having:
   both an X-ray powder diffraction pattern comprising peaks at 17.8 ± 0.2, 18.1 ± 0.2, 12.3 ± 0.2, 7.2 ± 0.2, 6.2 ± 0.2, 18.2 ± 0.2, 17.2 ± 0.2,16.8 ± 0.2, 22.1 ± 0.2 and 19.6 ± 0.2 degrees 2Θ when measured using CuKa radiation and
   a solid-state NMR spectrum comprising peaks at the following ¹⁹F chemical shifts expressed in parts per million: -112.5 ± 0.2, -116.1 ± 0.2 and -116.5 ± 0.2.
Clause 22. The crystalline form of the compound of formula (I) according to clause 16, characterized therein that it is an ethanol solvate form.
Clause 23. The crystalline ethanol solvate form of the compound of formula (I) according to clause 22, characterized therein that it is Form [I], having an X-ray powder diffraction pattern comprising peaks at 17.4 ± 0.2 and 19.3 ± 0.2 degrees 2Θ when measured using CuKa radiation.
Clause 24. The crystalline ethanol solvate form of the compound of formula (I) according to clause 22 or 23, characterized therein that it is Form [I],
   having an X-ray powder diffraction pattern comprising peaks at 17.4 ± 0.2, 19.3 ± 0.2, 11.5 ± 0.2, 10.5 ± 0.2, 9.3 ± 0.2 and 8.0 ± 0.2 degrees 2Θ when measured using CuKa radiation.
Clause 25. The crystalline form of the compound of formula (I) according to clause 16, characterized therein that it is Form [I],
   having a solid-state NMR spectrum comprising a peak at ¹⁹F chemical shift expressed in parts per million: -110 ± 0.2.
Clause 26. The crystalline form of the compound of formula (I) according to clause 25, characterized therein that it is Form [I],
   having a solid-state NMR spectrum comprising peaks at the following ¹³C chemical shifts expressed in parts per million: 56.6 ± 0.2, 47.2 ± 0.2 and 21.3 ± 0.2.
Clause 27. A process for preparing the crystalline compound Form [III] according to clause 17 to 21 said process comprising crystallization of Compound of formula (I) in n-butyl acetate (nBuOAc).
Clause 28. A process for preparing the crystalline compound Form [I] according to clause 22 to 25
   said process comprising the following steps:
      (a) suspending compound (I) Form [III] in ethanol over at least 8 hours,
      (b) filtrating the suspension,
      (c) washing the residue with ethanol and drying,
   resulting in the precipitation of Compound (I) Form [I] crystals.
Clause 29. A pharmaceutical composition comprising the crystalline compound Form [III] of formula (I): 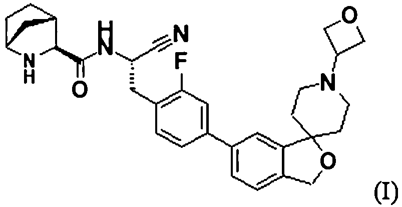
   and at least one pharmaceutically acceptable carrier or diluent,
   wherein the crystalline Form [III] has:
      (a) an X-ray powder diffraction pattern comprising peaks at 17.8 ± 0.2 and 18.1 ± 0.2 degrees 2Θ when measured using CuKa radiation; or
      (b) a solid-state NMR spectrum comprising peaks at the following ¹⁹F chemical shifts expressed in parts per million: -112.5 ± 0.2, -116.1 ± 0.2 and -116.5 ± 0.2, or
      (c) both at 17.8 ± 0.2 and 18.1 ± 0.2 degrees 2Θ when measured using CuKa radiation and a solid-state NMR spectrum comprising peaks at the following ¹⁹F chemical shifts expressed in parts per million: -112.5 ± 0.2, -116.1 ± 0.2 and -116.5 ± 0.2.
Clause 30. A pharmaceutical composition comprising the crystalline compound Form [I] of formula (I): 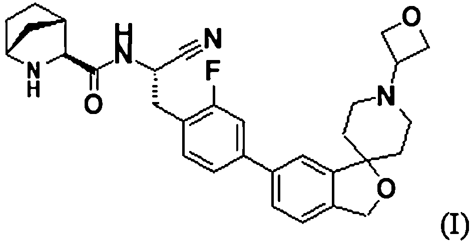
   and at least one pharmaceutically acceptable carrier or diluent,
   wherein the crystalline Form [I] has:
      (a) an X-ray powder diffraction pattern comprising peaks at 17.4 ± 0.2 and 19.3 ± 0.2 degrees 2Θ when measured using CuKa radiation.; or
      (b) a solid-state NMR spectrum comprising a peak at ¹⁹F chemical shift expressed in parts per million: -110 ± 0.2; or
      (c) both at 17.4 ± 0.2 and 19.3 ± 0.2 degrees 2Θ when measured using CuKa radiation and a solid-state NMR spectrum comprising a peak at ¹⁹F chemical shift expressed in parts per million: -110 ± 0.2.
Clause 31. Compound (I) for use in the treatment of a patient suffering from Hidradenitis suppurativa.
Clause 32. Compound (I) according to clause 31 for use in the treatment of a patient suffering from Hidradenitis suppurativa, characterized in that Compound (I) is administered in a daily dose of 2.5 mg.
Clause 33. Compound (I) according to clause 31 for use in the treatment of a patient suffering from Hidradenitis suppurativa, characterized in that Compound (I) is administered in a daily dose of 5 mg.
Clause 34. Compound (I) for use in the treatment of a patient suffering from ANCA-Associated Vasculitis.
Clause 35. Compound (I) according to clause 34 for use in the treatment of a patient suffering from ANCA-Associated Vasculitis, characterized in that Compound (I) is administered in a daily dose of 2.5 mg.
Clause 36. Compound (I) according to clause 34 for use in the treatment of a patient suffering from ANCA-Associated Vasculitis, characterized in that Compound (I) is administered in a daily dose of 5 mg.
Clause 37. A quasiquantitative method for the determination of neutrophil elastase (NE) activity in sputum, comprising the steps of:
   a) collecting sputum samples from patients;
   b) processing the sputum samples using Sputolysin^{®};
   c) preparing a NE substrate working solution comprising a fluorescent substrate specific to neutrophil elastase activity in TRIS-buffer pH 7.5;
   d) preparing a NE stop working solution specific to stop neutrophil elastase activity in TRIS-buffer pH 7.5;
   e) combining the sputum samples with the NE substrate working solution to initiate a fluorescence reaction and in parallel with the NE stop working solution for the determination of unspecific background fluorescence;
   f) incubating the mixture to allow the enzymatic reaction between neutrophil elastase and the fluorescent substrate under time and temperature adjusted conditions;
   g) determining the fluorescence of the samples using a fluorescence detection system; and
   h) comparing the total fluorescence with the unspecific background fluorescence.

The following items are part of the description:
Item 1. Crystalline form of the compound of formula (I) 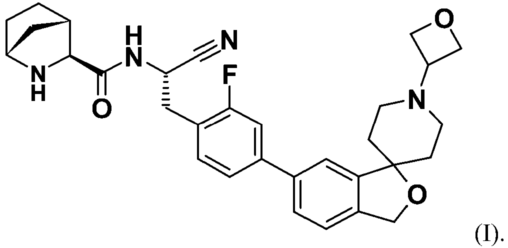
Item 2. The crystalline form according to item 1, wherein the Compound (I) is the free base in crystalline anhydrous form.
Item 3. The crystalline form according to any of the preceding items, wherein it is in Form [III], having an X-ray powder diffraction pattern comprising peaks at 17.8 ± 0.2 and 18.1 ± 0.2 degrees 2Θ when measured using CuKa radiation.
Item 4. The crystalline form according to any of the preceding items, wherein it is in Form [III], having an X-ray powder diffraction pattern comprising peaks at 17.8 ± 0.2, 18.1 ± 0.2, 12.3 ± 0.2, 7.2 ± 0.2 and 6.2 ± 0.2 degrees 2Θ when measured using CuKa radiation.
Item 5. The crystalline form according to any of the preceding items, wherein it is in Form [III], having a solid-state NMR spectrum comprising peaks at the following ¹⁹F chemical shifts expressed in parts per million: -112.5 ± 0.2, -116.1 ± 0.2 and -116.5 ± 0.2.
Item 6. The crystalline form according to any of the preceding items, wherein it is in Form [III], having a solid-state NMR spectrum comprising peaks at the following ¹³C chemical shifts expressed in parts per million: -85.2 ± 0.2, -47.3 ± 0.2 and -42.3 ± 0.2.
Item 7. The crystalline form according to any of the preceding items, wherein it is in Form [III], having an X-ray powder diffraction pattern comprising peaks at 17.8 ± 0.2, 18.1 ± 0.2, 12.3 ± 0.2, 7.2 ± 0.2, 6.2 ± 0.2, 18.2 ± 0.2, 17.2 ± 0.2,16.8 ± 0.2, 22.1 ± 0.2 and 19.6 ± 0.2 degrees 2Θ when measured using CuKa radiation.
Item 8. The crystalline form according to any of the preceding items, wherein it is in Form [III] obtained by or obtainable by the crystallization of Compound of formula (I) in n-butyl acetate (nBuOAc).
Item 9. A pharmaceutical composition comprising the crystalline form according to any of the preceding items and at least one pharmaceutically acceptable carrier or diluent.
Item 10. The pharmaceutical composition according to item 9, wherein the pharmaceutical composition is a film-coated tablet having a film coating and a tablet core, wherein the tablet core comprises the crystalline form of the compound of formula (I).
Item 11. The pharmaceutical composition according to item 10, wherein the film coating comprises an agent selected from the group consisting of hydroxypropyl methylcellulose, calcium carbonate, talc, polyethylene glycol, pigment, and combinations thereof; and the film coating is substantially free of titanium dioxide.
Item 12. A process for preparing a film-coated tablet comprising:
   (1) providing mannitol, Compound (I) Form [III] according to any of items 3 to 8, hydroxypropylcellulose, crospovidone and microcrystalline cellulose;
   (2) blending the ingredients from step (1), preferably in a diffusion mixer;
   (3) combining the resulting blend with magnesium stearate to make a final blend;
   (4) compressing the final blend into tablet cores, preferably using a rotary tablet press;
   (5) coating the tablets in a film coating suspension.
Item 13. A process for preparing the crystalline Compound (I) Form [III], according to any of items 3 to 8, said process comprising crystallization of Compound of formula (I) in n-butyl acetate (nBuOAc).
Item 14. The crystalline form according to any of items 1 to 8, or the pharmaceutical composition according to any of items 9 to 11, for use in the treatment or prevention of a pathology selected from the group consisting of bronchiectasis, NCFBE, CFBE, hidradenitis suppurativa, ANCA-associated vasculitis, idiopathic, post-infectious, primary ciliary dyskinesia, asthma and chronic obstructive pulmonary disease.
Item 15. The crystalline form, or the pharmaceutical composition, for use according to item 14, administered in a daily dose of 2.5 mg or 5 mg of crystalline form of the Compound (I).

### CLINICAL TRIALS

### AIRLEAF (1397-0012)

### DESCRIPTION OF THE CLINICAL TRIAL

This clinical trial was a multinational, randomised, double-blind, placebo-controlled, parallel-group, dose-finding clinical trial to investigate the efficacy, safety and tolerability of three different doses of Compound (I).

A history of symptoms consistent with bronchiectasis and a computed tomography scan consistent with the diagnosis are the main sources for the diagnosis for trial entry. Following a screening period of up to 6 weeks, participants were randomised in a 2:1:1:2 ratio to receive placebo or one of three once-daily doses of Compound (I) (1 mg, 2.5 mg and 5 mg). This was followed by a treatment period of at least 24 weeks and up to 48 weeks to obtain a sign of clinical efficacy of Compound (I) and a safety follow-up period of 4 weeks after the end of treatment. The treatment period concluded for all participants when the last randomised participant completed 24 weeks of treatment. Participants with early or permanent treatment discontinuation were asked to attend future visits as scheduled.

This duration of exposure also allowed assessment of possible effects on pulmonary exacerbations, lung function, patients reported outcomes and safety and tolerability of Compound (I).

Efficacy was assessed using pulmonary exacerbations, patient-reported outcomes (Quality of Life-Bronchiectasis Questionnaire [QoL-B], St. George's Respiratory Questionnaire [SGRQ] and spirometry for pulmonary function at every on-site visit (see Figure 1). Safety was monitored throughout the study.

The purpose of this study was to find out whether the Compound (I) helps people with bronchiectasis by preventing pulmonary exacerbations.

### PHARMACEUTICAL COMPOSITION

Compound (I) (at 1 mg, 2.5 mg and 5 mg) and matching placebo were administered as tablets (Table 1). Since the three doses were each reliant on a different tablet size, patients needed to take one tablet of each size per administration to maintain the blind.

Compound (I) containing tablets were administered in 3 dosage strengths (orally, qd): 1 mg (5.5 mm round), 2.5 mg (11x5.5 mm oval), 5 mg (14x6.8 mm oval). In addition to the drug substance, the tablets contained the following standard pharmaceutical excipients: mannitol, microcrystalline cellulose, hydroxypropylcellulose, crospovidone and magnesium stearate. Matching placebo tablets contained mannitol, microcrystalline cellulose, and magnesium stearate, i.e. placebo tablets, apart from being devoid of the drug substance, were also lacking the excipients hydroxypropyl cellulose and crospovidone, but were otherwise identical to verum tablets. The three dosage strengths were manufactured from a blend using milled drug substance. The manufacturing process used for these uncoated tablets consisted of sieving and blending steps followed by direct compression.

**Table 1: QUALITATIVE AND QUANTITATIVE COMPOSITION OF COMPOUND (I) TABLETS:**

| **Ingredients** | **Quantity in mg per tablet** | | | **Function** |
|---|---|---|---|---|
| | 1 mg | 2.5 mg | 5 mg | |
| Compound (I) | 1.00 | 2.50 | 5.00 | Active ingredient |
| Mannitol | 41.60 | 104.00 | 208.00 | Filler |
| Cellulose, microcrystalline | 20.80 | 52.00 | 104.00 | Filler |
| Hydroxypropyl cellulose | 2.00 | 5.00 | 10.00 | Binder |
| Crospovidone | 0.30 | 0.75 | 1.50 | Disintegrant |
| Magnesium stearate | 1.10 | 2.75 | 5.50 | Lubricant |
| Total (tablet) | 66.80 | 167.00 | 334.00 | |

### STUDY POPULATION

A total of 322 participants were randomised and received Compound (I) (1 mg [n=53], 2.5 mg [n=53] or 5 mg [n=107]) or placebo (n=109). A total of 295 participants (91.6%) completed the study. Exacerbations were assessed throughout the study. The participants performed lung function tests at study visits (FEV1 and FVC) and Quality of Life assessments (SGRQ and QoL-B) (Figure 1). The treatment effect in pulmonary exacerbations, in lung function tests and in Quality of Life assessments were compared between the "active agent groups" and the "placebo group".

### INCLUSION CRITERIA AND EXCLUSION CRITERIA:

Main inclusion and exclusion criteria are summarized in Table 2.

**Table 2: KEY INCLUSION AND EXCLUSION CRITERIA:**

| **Inclusion criteria** | **Exclusion criteria** |
|---|---|
| Male or female, aged ≥18 years* and ≤85 years at screening, adhering to contraception requirements. | AST and/or ALT >3 x ULN at Visit 1. |
| Signed and dated written informed consent prior to admission to the study, in accordance with Good Clinical Practice and local legislation. | Estimated glomerular filtration rate according to CKD-EPI formula <30 mL/min at Visit 1. |
| | Absolute blood neutrophil count <1000/mm³ at Visit 1. |
| Clinical history consistent with bronchiectasis (cough, chronic sputum production and/or recurrent respiratory infections) and investigator-confirmed diagnosis of bronchiectasis by CT scan. | |
| History of pulmonary exacerbations requiring antibiotic treatment. **In** the 12 months before Visit 1, patients must have had either: i) at least two exacerbations, or ii) one exacerbation and an SGRQ symptoms score of >40 at Screening Visit 1. | |
| Current sputum producers with a history of chronic expectoration who are able to provide a spontaneous sputum sample at Screening Visit 1. | Current diagnosis of cystic fibrosis, hy-pogammaglobulinaemia, common variable immunodeficiency, α1-antitrypsin deficiency, or allergic bronchopulmonary aspergillosis requiring treatment. |
| | Any clinically relevant acute respiratory infection or any other acute infection requiring systemic or inhaled anti-infective therapy. |
| | Any mycobacterial infections, including pulmonary non-tuberculous mycobacterial disease, currently being treated |
| | Severe periodontal disease (to be evaluated by a periodontist or dentist) or palmar kera-tosis. |
| | Any recent change in dose or regimen of maintenance bronchiectasis treatment. |
| | Any medical condition that could interfere with participation or conduct of the study. |

| | |
|---|---|
| *≥19 in Republic of Korea. †If allowed according to regulatory requirements. | |

ALT, alanine aminotransferase; AST, aspartate aminotransferase; CKD-EPI, Chronic Kidney Disease Epidemiology Collaboration; CT, computed tomography; SARS-CoV-2, severe acute respiratory syndrome coronavirus 2; SGRQ, ULN, upper limit of normal

### RANDOMIZATION.

Patients eligible for the trial based on the aforementioned criteria were randomised in a 2:1:1:2 ratio, respectively, to placebo once daily (n=109), Compound (I) 1 mg once daily (n=53), Compound (I) 2.5 mg once daily (n=53) or Compound (I) 5 mg once daily (n=107). The randomisation was stratified by *Pseudomonas aeruginosa* infection (yes/no) and by mac-rolide antibiotic maintenance (yes/no) at baseline.

All participants provided a sputum sample for central laboratory microbiological testing for *P. aeruginosa* at baseline.

### SAMPLE SIZE DETERMINATION.

The study was designed to demonstrate a non-flat, monotonic dose-response relationship on the time to first pulmonary exacerbation up to Week 48. The predefined dose-response shapes assumed a maximum of 50% reduction in the risk of experiencing an exacerbation (hazard ratio: 0.5) for Compound (I) 5 mg versus placebo. A total of 240 participants were needed to observe 134 events across all treatment arms, with a median time to first pulmonary exacerbation of 6.5 months for the placebo arm. This sample size, allocated with a 2:1:1:2 ratio, yielded a 90% power at a one-sided alpha level of 0.05 for the evaluation of the dose-finding part.

Due to a major increase in recruitment in the last 3 weeks of the recruitment phase, in combination with a decrease in the screening failure rate, a total of 322 participants were randomised into the trial (as per protocol, randomisation of all eligible screened people was allowed).

### TRIAL OBJECTIVES AND ENDPOINTS.

The primary objective was to demonstrate a non-flat dose-response relationship for the Compound (I) doses (1 mg, 2.5 mg and 5 mg) versus placebo on the primary endpoint, time to first pulmonary exacerbation up to Week 48. The secondary objective was to demonstrate superiority of Compound (I) 5 mg versus placebo on time to first pulmonary exacerbation up to Week 48, as well as on the key secondary endpoint, the rate of pulmonary exacerbations up to Week 48. Additional secondary endpoints were the absolute change from baseline at Week 24 in QoL-B respiratory symptoms domain score, SGRQ Symptoms score and post-bronchodilator per cent predicted forced expiratory volume in 1 second (ppFEV₁); relative change from baseline in sputum NE activity at Week 12; and the occurrence of an exacerbation by Week 24 (Table 3).

Safety was monitored throughout the study by assessment of adverse events (AEs), physical examination, monitoring of vital signs, safety laboratory parameters and 12-lead electrocardiogram. Skin examinations were performed by the investigator at each visit, and periodontal assessments were performed by a dentist or periodontist at screening and every 12 weeks during the treatment period. Potential severe drug-induced liver injury (DILI) was a protocol-defined AE of special interest (AESI) in this trial. Mode of action-related AEs included skin, periodontal and opportunistic infection AEs.

**Table 3: CLINICAL ENDPOINTS AND SAFETY ASSESSMENTS:**

| **Primary and key secondary clinical endpoints up to Week 48** | |
|---|---|
| • Time to first pulmonary exacerbation | |
| • Rate of pulmonary exacerbations | |

| **Secondary and further clinical endpoints up to Week 48** | |
|---|---|
| • Time to first severe pulmonary exacerbation* | |
| • Absolute change from baseline in post-bronchodilator ppFEV₁ | |
| • Absolute change from baseline in post-bronchodilator FEV₁ (mL) | |
| • Absolute change from baseline in post-bronchodilator ppFVC | |
| • Absolute change from baseline in post-bronchodilator FVC (mL) | |
| • Change from baseline in SGRQ symptoms score | |
| • Change from baseline in SGRQ total score | |
| • Change from baseline in QoL-B respiratory symptoms score | |

| **Secondary and further clinical endpoints up to Week 52** | |
|---|---|
| • Total duration of antibiotic treatment due to exacerbations | |

| **Safety assessments** | |
|---|---|
| • Physical examination | • 12-lead ECG |
| • Vital signs | • Periodontal assessments |
| • Safety laboratory parameters | • Skin assessments |

| | |
|---|---|
| *Severe pulmonary exacerbations were defined as those leading to hospitalisation and/or intravenous antibiotic administration, or with a fatal outcome. | |

Additional objectives of the trial were to confirm Compound (I)-mediated reduction of NE activity in sputum and evaluate pharmacokinetics, changes over time in blood and sputum bi-omarkers, safety and tolerability.

Neutrophil elastase activity in sputum has been measured by the fluorescence assay exemplified below.

### ASSAY FOR THE MEASUREMENT OF NEUTROPHIL ELASTASE ACTIVITY IN SPUTUM AND IN ZYMOSAN-STIMULATED BLOOD SAMPLES:

### Reagent / Buffer preparation

1. The NE-STOP WORKING solution (50µM MeOSuc-AAPV-CMK)
   a. One vial of 25mg lyophilizate MeOSuc-AAPV-CMK (NE stop peptide) is solved in 994µL DMSO. This stock solution (50mM) is aliquoted and the aliquots are stored frozen at -20°C for 12 months.
   b. On the day of the assay the NE-STOP WORKING solution (50µM MeOSuc-AAPV-CMK) is prepared about 10min before start of the assay by diluting the stock solution (50mM) with TRIS-buffer (100mM Tris/HCl, 1M NaCl buffer; pH 7.5) to the final concentration of 50µM MeOSuc-AAPV-CMK (NE-STOP WORKING solution).
2. The NE-Substrate WORKING solution (1mM MeOSuc-AAPV-AMC)
   a. To prepare the 100mM NE-Substrate STOCK solution one vial of MeOSuc-AAPV-AMC (NE substrate; 50mg) lyophilizate is solved in 796.6µL DMSO LoBind^{®} tubes. The solution is aliquoted (30µL per aliquot) and the aliquots are stored frozen at -20°C for 12 months.
   b. On the day of the assay directly before use the NE substrate STOCK solution (100mM) is diluted with TRIS-buffer (100mM Tris/HCl, 1M NaCl buffer; pH 7.5) to the final concentration of 1mM MeOSuc-AAPV-AMC (NE-Substrate WORKING solution) protected from light.

### Before the start of the assay:

a) 2h before the start of the assay TRIS-buffer (100mM Tris/HCl, 1M NaCl buffer; pH7.5) is equilibrated at room temperature.
b) Zymosan-stimulated human samples: 1h before the start of the assay the stimulated samples are thawed at room temperature, no movement. After thawing, the samples are centrifuged at 2000g for 5 minutes.
c) Sputum samples: The sputum sample is thawed at room temperature for about 15 minutes and stored on ice for further processing (e.g. 1:10 dilution in "NE Standard diluent"; ProAxsis).
d) 10min before the start of the assay, the 50µM NE-STOP working solution should be prepared.

### Assay Procedure

The assay is conducted in 96 well Optiplate^{™}-96 F, black plates: One sample is run on 2 different plates in parallel - on one plate the total fluorescence is measured ("Total") and on the second plate the unspecific background fluorescence is measured ("Background"). The only difference between both plates is the first solution added.
1. For the "Total" plate (Optiplate^{™}-96 F, black), 25µl of TRIS-buffer are pipetted into each well.
2. For the "Background" plate (Optiplate^{™}-96 F, black) - in parallel to the "Total" plate - 25µl of the NE-STOP WORKING solution (50µM; MeOSuc-AAPV-CMK) are pipetted into each well.
3. Immediately afterwards 50µL of the samples (undiluted zymosan-stimulated samples or diluted sputum samples) are added in duplicate wells using a 10-100µL pipette.
4. The plates are agitated for 10sec on a plate shaker (e.g.ThermoMixer^{®}) at 900rpm at room temperature.
5. The plates are then incubated on the benchtop for exactly 10 min at room temperature without movement (avoid direct sunlight).
6. During this incubation time the NE-Substrate working solution should be prepared.
7. Immediately afterwards 25µL of NE-Substrate WORKING solution (1.0mM; MeOSuc-AAPV-AMC) is added into each well on both plates.
8. The plates are agitated for 10sec on a plate shaker (e.g.ThermoMixer^{®}) at 900rpm at room temperature.
9. The plates are sealed with a sealing tape and then incubated for exactly 90min at 37°C in the dark on a plate shaker (e.g.ThermoMixer^{®}) without shaking (after 45 min of the incubation the fluorimeter has to be turned on).
10. At the end of the 90min incubation time, the plates are immediately measured in the fluorimeter/microplate reader. The order of both plates has to be kept the same during the entire study with the following settings:
   - Endpoint fluorescence measurement
   - Wavelength excitation: 360nm
   - Wavelength emission: 460nm

The final results are given in RFU = relative fluorescent units. The mean of the two dependent replicates per sample and CV is calculated using the software of the fluorimeter/microplate reader (e.g. SoftmaxPro or Magellan) for evaluation of the validity of the samples (CV<30%). The mean background fluorescence can be used as an internal quality control sample in addition to the quality control samples of the assay conduct. (In general, the CV over the study samples CV<10%).

The mean background fluorescence can be subtracted from the mean total fluorescence.

### Material / equipment:

| **Material / equipment name** | **Company** | **Model number / material number** |
|---|---|---|
| Analytical balance | Mettler Toledo | XS603S |
| Centrifuge | Eppendorf | Centrifuge 5430R |
| Manual pipettes | Rainin | |
| 10-100µL pipette | | Pipet-Lite XLS L-100 (17014384) |
| 100-1000µL pipette | | |
| 2-20mL | | Pipet-Lite XLS L-1000 (17014382) |
| | | Pipet-Lite XLS L-20mL (17014392) |
| Pipette tips | Rainin / Mettler Toledo | GP LTS 200µL (30389276) |
| | | GP LTS 1000µL (30389272) |
| | | RC LTS 20mL (17001131) |
| HandyStep^{®} AutoRep E | Rainin / Brand | |
| HandyStep^{®}, PD-Tips II | Brand | PD-Tips II 1.0mL (705704) |
| | | PD-Tips II 2.5mL (705708) |
| | | PD-Tips II 5.0mL (705710) |
| | | PD-Tips II 10.0mL (705712) |
| Reaction tubes | Eppendorf | |
| 2mL LoBind^{®} tubes | | 30108132 |
| 5mL LoBind^{®} tubes | | 30122356 |
| Sealing tapes, Polypropylene | HJ-Bioanalytik GmbH | 900 510 |
| Microtiter plates, Optiplate^{™}-96 F, Black | Perkin Elmer | 6005270 |
| pH-Meter | Schott instruments | |
| ThermoMixer^{®} Eppendorf C | Eppendorf | 5382000015 |
| + SmartBlock Plates | | 5363000039 |
| Fluorimeter, (Microplate reader) | Molecular Devices | MaxLine series M2 |
| Refrigerator | Liebherr | |
| Freezer | Liebherr | |

### Reagent / Buffer:

| **Reagent** | **Description (manufacturer, product number)** |
|---|---|
| TRIS-HCl | Sigma-Aldrich (Cat No: T5941) |
| | CAS-No: 1185-53-1, |
| | M: 157.6 g/mol |
| NaCl | Merck (Cat No: 1.06404) |
| | CAS-No: 7647-14-5, |
| | M: 58.44 g/mol |
| NaOH (1 M) | Merck (Cat No: 1.09137) |
| | CAS-No: 1310-73-2 |
| Dimethylsulfoxide (DMSO) | Merck (Cat No: D8418) |
| | CAS: 67-68-5 |
| MeOSuc-AAPV-AMC (NE substrate) | Bachem (Cat No: 4005227) |
| | CAS: 72252-90-5 |
| | M: 627.7 g/mol |
| MeOSuc-AAPV-CMK (NE stop peptide) | Bachem (Cat No: 4009927) |
| | CAS: 65144-34-5 |
| | M: 503 g/mol |
| Millipore H2O | Internally produced Millipore water |

### Zymosan-stimulation of human citrate blood

### Zymosan A suspension (10mg/mL) preparation

Weigh zymosan A powder directly in a falcon tube and add 0.9% NaCl (phys. NaCl) buffer to a final concentration of 10mg/mL. The Zymosan suspension is homogenized with a UltraTurrax for 1min, followed by 1min with a vortex. The suspension is stable at 4°C for 1 week. The actual volume of Zymosan suspension used should be stored at room temperature for 1 hour before use for equilibration.

### Stimulation procedure

1. Na-citrate blood is collected and directly after collection the blood drawing tube should be inverted 10 times to prevent clotting.
2. Directly afterwards allow the citrated blood to stand for exactly 60 minutes without movement on the benchtop avoid exposure to direct sunlight. This step is critical! In case the 60min cannot be kept, up to + 10min may still be valid, but the quality of the study data depends on the accuracy in this step.
3. During this first incubation step, the centrifuge should be cooled down to 4°C.
4. Shortly before the end of the 60min incubation time the zymosan suspension (10mg/mL) should be mixed well for 1min with a shaker/vortex, as zymosan sediments very quickly. Additionally, it is important that the suspension used has reached room temperature.
5. Immediately after the 60min incubation step the zymosan suspension (10mg/mL) is added to the citrated blood to a final concentration of 0.91 mg/mL. The tube is mixed by inverting the tube 10 times.
6. Immediately afterwards the tube is incubated on the benchtop for exactly 80 min without movement, avoid exposure to direct sunlight. This step is critical! In case the 80min cannot be kept, up to + 5 min still valid, but the quality of the study data depends on the accuracy in this step.
7. The incubation/stimulation is stopped by centrifugating the tube at 1000g, for 15min, at 4°C.
8. After the centrifugation, the zymosan-stimulated citrate plasma is gained, by transferring the supernatant above the buffy coat into a fresh tube. In this fresh tube, the plasma should be mixed (vortex for 10sec at 3000rpm) before it is aliquoted and stored at -20°C.
9. The Zymosan-stimulated citrate plasma can be stored at -20°C for 6 months.

### Material / equipment:

| **Material / equipment name** | **Company** | **Model number / material number** |
|---|---|---|
| S-Monovette, Na-citrate | Sarstedt | S-Monovette, Tri-Sodiumcitrate 3.2%, 5mL |
| Analytical balance | Mettler Toledo | XS603S |
| Centrifuge | Heraeus | Multifuge 3SR+ |
| Manual pipettes 100-1000µL pipette 2-20mL | Rainin | Pipet-Lite XLS L-1000 (17014382) |
| | | Pipet-Lite XLS L-20mL (17014392) |
| Pipette tips | Rainin / Mettler Toledo | GP LTS 1000µL (30389272) |
| | | RC LTS 20mL (17001131) |
| HandyStep^{®} AutoRep E | Rainin / Brand | |
| HandyStep^{®}, PD-Tips II | Brand | PD-Tips II 10.0mL (705712) |
| Protein LoBind^{®} conical tube 15mL or 50mL | Eppendorf | 15mL (0030122.216) |
| | | 50mL (0030122.240) |
| Homogenizer | IKA | T10 basic |
| Shaker / Vortex | Heidolph | Reax top |

### Reagent / Buffer:

| **Reagent** | **Description (manufacturer, product number)** |
|---|---|
| Zymosan A from *Saccharomyces cerevisiae* | Sigma-Aldrich (Cat No. Z4250) |
| | CAS-No: 58856-93-2 |
| NaCl | Merck (Cat No: 1.06404) |
| | CAS-No: 7647-14-5, |
| | M: 58.44 g/mol |
| Millipore H2O | Internally produced Millipore water |

### Sputum processing for NE activity measurement

### Reagent / Buffer preparation

Dilute Sputolysin^{®} reagent (1% DTT) 1:10 in 1xPBS buffer and mix for about 10sec with a shaker/vortex. The 1xPBS/0.1% DTT buffer is used for the homogenization of sputum plugs. It should be prepared on the same day of use and can be stored at room temperature until use. Sputum processing
1. The centrifuge should be cooled down to 4°C, in preparation for the centrifugation of the sputum sample.
2. The sputum sample is thawed at room temperature for 15 minutes and stored on ice for further processing.
3. In preparation for the subsequent steps, the weight of a 15mL vial is determined.
4. After ensuring that the sputum sample is completely thawed, the entire sputum plug is transferred into the 15mL vial.
5. Determine the net weight of the sputum plug and add 4 mL of 1xPBS/0.1% DTT buffer per g of sputum plug: For example: 1g sputum + 4mL PBS/0.1% DTT buffer
6. The buffer should be at room temperature.
7. The 15mL vial including the sputum plug and PBS/0.1% DTT buffer is mixed on a Shaker / Vortex for 10sec at 3000rpm.
8. Immediately afterwards, the sample is manually homogenized in case of a tight sputum plug: The sample is repeatedly aspirated up and down through a 23-gauge needle on a syringe. This step is repeated 4 times per gram of sputum. Depending on the patient population you can use this as a general step.
9. Immediately afterwards, the sample is mixed for exactly 30 minutes on an HulaMixer^{™} at 37°C in the 37°C incubation hood or in a moving water bath at 37°C.
10. After that incubation the sample is centrifuged at 3000g for 30 minutes at 4°C.
11. The supernatant is collected into a fresh 15mL LoBind^{®} vial on ice. This supernatant solution is mixed again (Vortex, 10sec at 3000rpm).
12. Finally, the sputum supernatant is aliquoted into 2mL pre labelled LoBind^{®} tubes on ice.
13. The sputum supernatant is stored at -80°C for 12 months.
14. It is strongly recommended to dilute the sputum supernatant directly before the measurement in "NE Standard diluent"; ProAxsis and not to do the dilution before freezing.

### Material / equipment:

| **Material / equipment name** | **Company** | **Model number / material number** |
|---|---|---|
| Analytical balance | Mettler Toledo | XS603S |
| Centrifuge | Heraeus | Multifuge 3SR+ |
| Manual pipettes 100-1000µL pipette 2-20mL | Rainin | Pipet-Lite XLS L-1000 (17014382) |
| | | Pipet-Lite XLS L-20mL (17014392) |
| Pipette tips | Rainin / Mettler Toledo | GP LTS 1000µL (30389272) |
| | | RC LTS 20mL (17001131) |
| Syringe, sterile | B. Braun | 5mL or 10mL |
| Needle, sterile | B. Braun | 23G |
| HandyStep^{®} AutoRep E | Rainin / Brand | |
| HandyStep^{®}, PD-Tips II | Brand | PD-Tips II 10.0mL (705712) |
| Protein LoBind^{®} conical tube 15mL or 50mL | Eppendorf | 15mL (0030122.216) |
| | | 50mL (0030122.240) |
| Shaker / Vortex | Heidolph | Reax top |
| Sample mixer | ThermoFisher Scientific | HulaMixer^{™} (Cat. No. 15920D) |
| Incubation hood | | Not specified |
| Water bath (agitated) | | Not specified |

### Reagent / Buffer:

| **Reagent** | **Description (manufacturer, product number)** |
|---|---|
| Sputolysin^{®} Reagent | Calbiochem / Millipore (Cat. No. 560000-1SET) |
| | CAS-No: 3483-12-3 |
| NaCl | Merck (Cat No: 1.06404) |
| | CAS-No: 7647-14-5, |
| | M: 58.44 g/mol |
| Na2HPO4*2H2O | Merck (Cat No: 1.06580) |
| | CAS-No: 10028-24-7 |
| | M: 178 g/mol |
| KH2PO4 | Merck (Cat No: 1.04873) |
| | CAS-No: 7778-77-0 |
| | M: 136 g/mol |
| Millipore H2O | Internally produced Millipore water |
| NE Standard diluent | ProAxsis (Cat. No. PA010) |

### STATISTICAL ANALYSES

### Statistical analyses for primary objective (dose response relationship) and secondary objective (clinical primary and key secondary endpoint):

For the evaluation of the dose-response relationship, a multiple comparison procedure with modelling techniques (MCP-Mod) [ Pinheiro J, Bornkamp B, Glimm E, Bretz F. Model-based dose finding under model uncertainty using general parametric models. Stat Med 2014: 33(10): 1646-1661.] was used. This hybrid approach, which combines hypothesis testing and modelling, has been recognised by regulatory authorities (European Medicines Agency in 2014 and the U.S. Food & Drug Administration in 2016) as an appropriate statistical method for Phase II dose-finding trials [ European Medicines Agency (EMA) "Qualification Opinion of MCP-Mod as an efficient statistical methodology for model-based design and analysis of Phase II dose finding studies under model uncertainty" (2014) ; USA Food and Drug Administration (FDA) Statistical review and evaluation qualification of statistical approach: MCP-Mod. 2016. ]. MCP-Mod allowed for the simultaneous evaluation of predefined possible dose-response models, and the identification of the best-fitting model or subset of models, while keeping full control of the type I error at 0.05, one-sided. A hierarchical testing strategy was applied whereby if a dose-response relationship was established, a pairwise comparison to assess the benefit of Compound (I) 5 mg versus placebo on the time to first pulmonary exacerbation up to Week 48, followed by the rate of pulmonary exacerbations at Week 48, would be performed each at a one-sided 2.5% significance level. The time to first pulmonary exacerbation was analysed with the use of a Cox proportional hazards regression model, which included the categorical effect of treatment and the stratification factors (*P. aeruginosa* status and maintenance use of macrolides) at baseline as covariates. A Kaplan-Meier plot was also provided by treatment. The rate of pulmonary exacerbations was analysed using a negative binomial model, which included the fixed, categorical effects of treatment, the stratification factors at baseline, and the logarithm of the duration of follow-up as an offset. The evaluation of Compound (I) 2.5 mg or 1 mg versus placebo on time to first pulmonary exacerbation or rate of pulmonary exacerbations was considered exploratory. Participants with early treatment discontinuation were followed up for exacerbations, and all data collected up to Week 48 were used in the analysis of the primary and key secondary endpoint.

### Statistical analyses for secondary and further endpoints

Absolute change from baseline at Week 24 for the Quality of Life-Bronchiectasis (QoL-B) respiratory symptoms domain score and the St. George's Respiratory Questionnaire (SGRQ) Symptoms score and Total score was analysed using a restricted maximum likelihood (REML)-based analysis of covariance model. The model included treatment and the stratification factors at baseline as discrete fixed effects, and baseline QoL-B respiratory symptoms domain score, SGRQ Symptoms score or SGRQ Total score as the continuous fixed effect. Least-squares mean and corresponding 95% confidence intervals (CIs) were provided by treatment group.

The absolute change from baseline in lung function parameters was analysed using an REML-based approach using a mixed model with repeated measurements (MMRM). The model included the fixed, categorical effects of treatment at each visit, the stratification factors, and the fixed continuous effects of baseline lung function parameters at each visit.

The proportion of participants with an exacerbation at predefined time points was based on a Kaplan-Meier (KM) analysis. KM survival probability at each time point was compared between each Compound (I) dose and placebo, and the variance of each KM estimate was calculated using the Greenwood's formula. The time to first severe exacerbation up to Week 48 was analysed in a similar way to the analysis of the primary endpoint.

The duration of antibiotic treatment due to pulmonary exacerbations, expressed as number of days per year, was computed for each participant. Summary statistics were provided per treatment group. Comparisons between each Compound (I) dose and placebo were based on two sample t-tests.

### Post hoc responder analysis

The percentage of participants with a response defined as an ≥8-point change from baseline at Week 24 (or Week 48) in QoL-B respiratory symptoms domain score was computed. For SGRQ Total score, a responder was defined as having a having a ≥4-point decrease from baseline. Differences in percentages between each Compound (I) dose and placebo were derived along with their respective 95% CIs based on the Newcombe method.

### RESULTS ON EFFICACY AND SAFETY ENDPOINTS

### Dose response

The dose-response analysis revealed a significant dose-dependent benefit of Compound (I) over placebo on the time to first pulmonary exacerbation up to Week 48 (shape: Emax 1; adjusted p=0.0448) (Figure 2). The MCP-Mod model-predicted hazard ratios (95% confidence intervals [CIs]) for Compound (I) 1 mg, 2.5 mg and 5 mg versus placebo were 0.85 (0.58 to 1.25), 0.75 (0.51 to 1.10) and 0.68 (0.47 to 1.00), respectively.

### Pulmonary exacerbations

Assessment of the secondary objective (the pairwise comparison of Compound (I) 5 mg versus placebo on the time to first pulmonary exacerbation up to Week 48) revealed a 29% reduction in risk favouring Compound (I) 5 mg (adjusted hazard ratio: 0.71; 95% CI 0.48 to 1.05; p=0.0857). The effect for Compound (I) 2.5 mg with placebo was 0.66 (0.40 to 1.08). The effect for Compound (I) 1 mg was 0.93 (0.60 to 1.45) (Figure 3 and Table 4).

**Table 4: COX REGRESSION MODEL OF TIME TO FIRST PULMONARY EXACERBATION UP TO WEEK 48.**

| | | **Placebo** | **1 mg** | **2.5 mg** | **5 mg** |
|---|---|---|---|---|---|
| Number of patients in analysis set, N (%) | | 109 (100.0) | 53 (100.0) | 53 (100.0) | 107 (100.0) |

| Time to first pulmonary exacerbation up to 48 weeks | | | | | |
|---|---|---|---|---|---|
| Number of patients with event, N (%) | | 59 (54.1) | 29 (54.7) | 21 (39.6) | 44 (41.1) |
| Time to event [weeks]¹ - 25^{th} percentile | | 14.4 | 15.1 | 15.0 | 13.7 |
| (95% CI) | | (10.4, 17.6) | (10.3, 20.0) | (6.6, 31.4) | (6.7, 23.6) |
| Time to event [weeks]¹ - median | | 29.6 | 31.7 | not reached | not reached |
| (95% CI) | | (23.0, 37.6) | (20.0, n.e.) | (23.6, n.e.) | (30.3, n.e.) |

| Comparison vs. placebo | | | | | |
|---|---|---|---|---|---|
| | Hazard ratio² (95% CI) | | 0.93 (0.60, 1.45) | 0.66 (0.40, 1.08) | 0.71 (0.48, 1.05) |
| | p-value (nominal) | | 0.7483 | 0.0988 | 0.0857 |

The results above were generally consistent across several predefined subgroups.

Across all three doses of Compound (I), the hazard ratios were similar or lower in the subgroup of participants who had one exacerbation in the past year with a high symptom burden versus participants who had two exacerbations in the past year (Figure 11,12,13).

Compound (I), 5 mg and 2.5 mg showed a numerical improvement in pulmonary exacerbation rate up to Week 48. The adjusted event rate ratio (95% CIs) for Compound (I) 5 mg was 0.89 (0.63 to 1.26). The adjusted event rate ratio (95% CIs) was better for Compound (I) 2.5 mg [0.68 (0.43 to 1.08)]. Compound (I) 1 mg had a rate ratio of 1.03 (0.69 to 1.56) (Figure 4 and Table 5).

**Table 5: NEGATIVE BINOMIAL REGRESSION MODEL OF RATE OF PULMONARY EXACERBATIONS UP TO WEEK 48.**

| | | **Placebo** | **1 mg** | **2.5 mg** | **5 mg** |
|---|---|---|---|---|---|
| Number of patients in analysis set, N (%) | | 109 (100.0) | 53 (100.0) | 53 (100.0) | 107 (100.0) |

| Pulmonary exacerbations up to 48 weeks, N (%) | | | | | |
|---|---|---|---|---|---|
| | 0 | 50 (45.9) | 24 (45.3) | 32 (60.4) | 63 (58.9) |
| | 1 | 40 (36.7) | 18 (34.0) | 14 (26.4) | 27 (25.2) |
| | 2 | 12 (11.0) | 8 (15.1) | 6 (11.3) | 10 (9.3) |
| | ≥3 | 7 (6.4) | 3 (5.7) | 1 (1.9) | 7 (6.5) |
| Total number of events | | 85 | 44 | 29 | 75 |
| Total observational time (patient years) | | 75.0 | 37.4 | 37.3 | 73.4 |
| Unadjusted event rate (events per 1 patient year) | | 1.13 | 1.18 | 0.78 | 1.02 |
| Adjusted¹ event rate (events per 1 patient year) | | 1.25 | 1.30 | 0.85 | 1.11 |
| | 95% CI | (0.96, 1.63) | (0.91, 1.86) | (0.56, 1.28) | (0.84, 1.47) |

| Comparison vs. placebo | | | | | |
|---|---|---|---|---|---|
| | Adjusted¹ event rate ratio (SE) | | 1.034 (0.218) | 0.677 (0.160) | 0.886 (0.158) |
| | 95% CI | | (0.685, 1.563) | (0.426, 1.076) | (0.626, 1.256) |
| | p-value (nominal) | | 0.8725 | 0.0987 | 0.4980 |

The risk of experiencing a pulmonary exacerbation in the first 24 weeks versus placebo was lower in the Compound (I) 2.5 mg and 5 mg groups (risk difference [95% CI] 2.5 mg: -5.7% [-21.6% to 10.3%]; 5 mg: -7.1% [-20.1% to 5.9%]) compared with the Compound (I) 1 mg group (2.7% [-13.7% to 19.0%]) (Figure 5 and Table 6).

**Table 6: RISK OF EXPERIENCING A PULMONARY EXACERBATION -**

| **Participants at risk n/N (%)** | **Week 12** | **Week 24** | **Week 36** | **Week 48** |
|---|---|---|---|---|
| Placebo | 89/109 (81.7) | 59/109 (54.1) | 24/109 (22.0) | 9/109 (8.3) |
| Compound (I) 1 mg | 41/53 (77.4) | 28/53 (52.8) | 15/53 (28.3) | 7/53 (13.2) |
| Compound (I) 2.5 mg | 41/53 (77.4) | 34/53 (64.2) | 17/53 (32.1) | 8/53 (15.1) |
| Compound (I) 5 mg | 82/107 (76.6) | 65/107 (60.7) | 34/107 (31.8) | 11/107 (10.3) |

Compound (I) 2.5 mg reduced the risk of experiencing a severe pulmonary exacerbation within 48 weeks compared with placebo (hazard ratio: 0.12; 95% CI 0.02 to 0.95), whereas no treatment benefit was observed for Compound (I) 1 mg or Compound (I) 5 mg (Table 7, Figure 6).

**Table 7: TIME TO FIRST SEVERE PULMONARY EXACERBATIONS UP TO WEEK 48**

| | | **Placebo** | **1 mg** | **2.5 mg** | **5 mg** |
|---|---|---|---|---|---|
| Number of patients in analysis set, N (%) | | 109 (100) | 53 (100) | 53 (100) | 107 (100) |

| Time to first severe pulmonary exacerbation up to 48 weeks | | | | | |
|---|---|---|---|---|---|
| | Number of patients with event, N (%) | 15 (13.8) | 6 (11.3) | 1 (1.9) | 11 (10.3) |
| | Median time to event (weeks)¹ | Not reached | Not reached | Not reached | Not reached |
| | 95% CI | (n.e, n.e) | (n.e, n.e) | (n.e, n.e) | (n.e, n.e) |

| Comparison vs. placebo | | | | | |
|---|---|---|---|---|---|
| | Hazard ratio² | | 0.79 | 0.12 | 0.73 |
| | (95% CI) | | (0.31, 2.04) | (0.02, 0.95) | (0.34, 1.59) |
| | p-value (nominal) | | 0.6260 | 0.0440 | 0.4310 |

The mean annual duration of antibiotic treatment due to pulmonary exacerbations was numerically shorter in the Compound (I) 2.5 mg group (mean±standard deviation: 20.6±12.7 days/year) compared with placebo (38.8±38.7 days/year). The mean duration of treatment for the Compound (I) 5 mg and 1 mg dose groups was 32.1±32.1 days/year and 39.0±37.3 days/year, respectively (Table 8).

**Table 8: DURATION OF ANTIBIOTIC TREATMENT DUE TO PULMONARY EXACERBATIONS UP TO WEEK 52.**

| | | **Placebo** | **1 mg** | **2.5 mg** | **5 mg** |
|---|---|---|---|---|---|
| Number of patients (N, %) | | 109 (100%) | 53 (100%) | 53 (100%) | 107 (100%) |

| Any antibiotic treatment (N, %) | | | | | |
|---|---|---|---|---|---|
| | N | 109 (100%) | 53 (100%) | 53 (100%) | 107 (100%) |
| | No | 42 (38.5%) | 20 (37.7%) | 27 (50.9%) | 51 (47.7%) |
| | Yes | 67 (61.5%) | 33 (62.3%) | 26 (49.1%) | 56 (52.3) |

| Duration of antibiotic treatment (days/year) up to Week 52 | | | | | |
|---|---|---|---|---|---|
| | N | 67 | 33 | 26 | 56 |
| | Mean | 38.8 | 39.0 | 20.6 | 32.1 |
| | SD | 38.7 | 37.3 | 12.7 | 32.1 |

In summary a clinical meaningful reduction of the exacerbation risk is shown in Figure 3. There is a reduction of the exacerbation risk by 34% at the 2.5 mg dose and a reduction of the exacerbation risk by 29 % at the 5 mg dose. In contrast to the reasonably expected best effect at the 5 mg dose the most advantageous effect is demonstrated at the lower 2.5 mg dose.

A clinical meaningful effect on the annualized exacerbation rate could be demonstrated for the 5 mg and the 2.5 mg dose (Figure 4). The annualized exacerbation rate was reduced by 32 % at the 2.5 mg and by 11 % at the 5 mg dose. In contrast to the reasonably expected best effect at the 5 mg dose the most advantageous effect is demonstrated at the lower 2.5 mg dose.

A clinical meaningful reduction of the severe pulmonary exacerbation risk is shown in Figure 6. There is a reduction of an exacerbation risk by 88% at the 2.5 mg dose and a reduction of an exacerbation risk by 27 % at the 5 mg dose. In contrast to the reasonably expected best effect at the 5 mg dose the most advantageous effect is demonstrated at the lower 2.5 mg dose.

### Lung function

The Compound (I) 2.5 mg dose led to the greatest change from baseline in FEV₁ in mL at Week 24 (adjusted mean versus placebo: 52.41 mL; 95% CI -13.80 to 118.63) (Table 9). This effect of the Compound (I) 2.5 mg dose was nominally similar at Week 36 (78.41 mL; 95% CI 12.17 to 144.65) and Week 48 (47.73 mL; 95% CI -54.94 to 150.40). The results for ppFEV₁ were similar to those for FEV₁ absolute values (Table 9, Figure 7).

Regarding forced vital capacity (FVC), the Compound (I) 2.5 mg dose led to the greatest change from baseline in FVC in mL at Week 24 (adjusted mean versus placebo: 80.11 mL; 95% CI -4.88 to 165.10), with even more pronounced effects at Week 36 (116.73 mL; 95% CI 29.90 to 203.57) and Week 48 (132.0 mL; 95% CI -7.64 to 271.64). The results for ppFVC were similar to those for FVC absolute values (Table 10, Figure 8).

**Table 9: ABSOLUTE CHANGE FROM BASELINE IN FEV1 (IN ML AND PER CENT PREDICTED) AT WEEKS 24, 36 AND 48.**

| | **Placebo** | **Compound (I)** | | |
|---|---|---|---|---|
| | | **1 mg** | **2.5 mg** | **5 mg** |
| **FEV₁ in mL** | | | | |
| **Week 24** | | | | |
| Number of participants | 97 | 50 | 49 | 86 |
| Absolute change from baseline - adjusted mean (95% CI) | -20.03 (-58.43 to 18.38) | 2.86 (-50.64 to 56.35) | 32.39 (-21.54 to 86.31) | -13.43 (-53.78 to 26.92) |
| Comparison versus placebo - adjusted mean (95% CI) | - | 22.89 (-42.92 to 88.69) | 52.41 (-13.80 to 118.63) | 6.60 (-49.14 to 62.34) |

| **Week 36** | | | | |
|---|---|---|---|---|
| Number of participants | 62 | 35 | 32 | 61 |
| Absolute change from baseline - adjusted mean (95% CI) | -67.21 (-105.75 to -28.68) | -14.95 (-66.92 to 37.03) | 11.20 (-42.55 to 64.94) | -46.55 (-85.87 to - 7.24) |
| Comparison versus placebo - adjusted mean (95% CI) | - | 52.27 (-12.33 to 116.86) | 78.41 (12.17 to 144.65) | 20.66 (-34.51 to 75.83) |

| **Week 48** | | | | |
|---|---|---|---|---|
| Number of participants | 33 | 18 | 22 | 37 |
| Absolute change from baseline - adjusted mean (95% CI) | -48.00 (-111.32 to 15.32) | 9.95 (-76.53 to 96.42) | -0.27 (-81.13 to 80.60) | -81.86 (-143.89 to - 19.84) |
| Comparison versus placebo - adjusted mean (95% CI) | - | 57.94 (-49.28 to 165.17) | 47.73 (-59.94 to 150.40) | -33.87 (-122.45 to 54.71) |

| **ppFEV₁** | | | | |
|---|---|---|---|---|
| **Week 24** | | | | |
| Number of participants | 97 | 50 | 49 | 86 |
| Absolute change from baseline - adjusted mean (95% CI) | -1.05 (-2.38 to 0.28) | -0.14 (-2.00 to 1.71) | 1.16 (-0.71 to 3.03) | -0.90 (-2.30 to 0.50) |
| Comparison versus placebo - adjusted mean (95% CI) | - | 0.90 (-1.38 to 3.19) | 2.21 (-0.09 to 4.51) | 0.15 (-1.79 to 2.08) |

| **Week 36** | | | | |
|---|---|---|---|---|
| Number of participants | 62 | 35 | 32 | 61 |
| Absolute change from baseline - adjusted mean (95% CI) | -2.40 (-3.79 to - 1.01) | -0.58 (-2.45 to 1.29) | 0.39 (-1.54 to 2.33) | -1.63 (-3.05 to - 0.21) |
| Comparison versus placebo - adjusted mean (95% CI) | - | 1.82 (-0.51 to 4.15) | 2.79 (0.41 to 5.17) | 0.77 (-1.21 to 2.75) |

| **Week 48** | | | | |
|---|---|---|---|---|
| Number of participants | 33 | 18 | 22 | 37 |
| Absolute change from baseline - adjusted mean (95% CI) | -2.20 (-4.32 to - 0.09) | 0.00 (-2.88 to 2.88) | -0.23 (-2.93 to 2.47) | -2.59 (-4.66 to - 0.52) |
| Comparison versus placebo - adjusted mean (95% CI) | - | 2.20 (-1.37 to 5.78) | 1.97 (-1.46 to 5.40) | -0.39 (-3.35 to 2.57) |

| | | | | |
|---|---|---|---|---|
| *Estimates were obtained from a mixed model for repeated measures, with *P. aeruginosa* status at baseline and maintenance use of macrolides at baseline as categorical fixed effects, interactions terms for treatment by visit and baseline by visit, and unstructured covariance matrix structure for repeated measurements within participants. | | | | |

**Table 10: ABSOLUTE CHANGE FROM BASELINE IN FVC (IN ML AND PER CENT PREDICTED) AT WEEKS 24, 36 AND 48.**

| | **Placebo** | **Compound (I)** | | |
|---|---|---|---|---|
| | | **1 mg** | **2.5 mg** | **5 mg** |
| **FVC in mL** | | | | |
| **Week 24** | | | | |
| Number of participants | 96 | 50 | 49 | 89 |
| Absolute change from baseline - adjusted mean (95% CI) | -33.85 (-83.36 to 15.66) | -4.20 (-72.74 to 64.35) | 46.26 (-22.89 to 115.41) | -16.70 (-67.86 to 34.46) |
| Comparison versus placebo - adjusted mean (95% CI) | - | 29.66 (-54.83 to 114.14) | 80.11 (-4.88 to 165.10) | 17.16 (-54.16 to 88.47) |

| **Week 36** | | | | |
|---|---|---|---|---|
| Number of participants | 62 | 35 | 32 | 62 |
| Absolute change from baseline - adjusted mean (95% CI) | -71.64 (-122.37 to -20.91) | -22.70 (-90.40 to 45.00) | 45.10 (-25.21 to 115.41) | -52.93 (-103.91 to - 1.95) |
| Comparison versus placebo - adjusted mean (95% CI) | - | 48.94 (-35.48 to 133.36) | 116.73 (29.90 to 203.57) | 18.71 (-53.61 to 91.03) |

| **Week 48** | | | | |
|---|---|---|---|---|
| Number of participants | 33 | 18 | 22 | 39 |
| Absolute change from baseline - adjusted mean (95% CI) | -49.20 (-135.71 to 37.31) | -41.33 (-158.74 to 76.07) | 82.80 (-26.90 to 192.50) | -131.10 (-213.53 to - 48.66) |
| Comparison versus placebo - adjusted mean (95% CI) | - | 7.86 (-137.99 to 153.72) | 132.00 (-7.64 to 271.64) | -81.90 (-201.37 to 37.57) |

| **ppFVC** | | | | |
|---|---|---|---|---|
| **Week 24** | | | | |
| Number of participants | 96 | 50 | 49 | 89 |
| Absolute change from baseline - adjusted mean (95% CI) | -1.31 (-2.69 to 0.07) | -0.13 (-2.04 to 1.78) | 1.59 (-0.33 to 3.52) | -0.62 (-2.04 to 0.81) |
| Comparison versus placebo - adjusted mean (95% CI) | - | 1.18 (-1.17 to 3.54) | 2.90 (0.54 to 5.27) | 0.69 (-1.29 to 2.68) |

| **Week 36** | | | | |
|---|---|---|---|---|
| Number of participants | 62 | 35 | 32 | 62 |
| Absolute change from baseline - adjusted mean (95% CI) | -2.11 (-3.55 to - 0.66) | -0.57 (-2.50 to 1.37) | 1.19 (-0.82 to 3.20) | -1.33 (-2.79 to 0.13) |
| Comparison versus placebo - adjusted mean (95% CI) | - | 1.54 (-0.87 to 3.96) | 3.30 (0.83 to 5.78) | 0.78 (-1.29 to 2.84) |

| **Week 48** | | | | |
|---|---|---|---|---|
| Number of participants | 33 | 18 | 22 | 39 |
| Absolute change from baseline - adjusted mean (95% CI) | -1.89 (-4.14 to 0.36) | -1.04 (-4.09 to 2.01) | 2.19 (-0.65 to 5.03) | -3.28 (-5.42 to -1.14) |
| Comparison versus placebo - adjusted mean (95% CI) | - | 0.85 (-2.94 to 4.65) | 4.09 (0.46 to 7.71) | -1.39 (-4.50 to 1.73) |

| | | | | |
|---|---|---|---|---|
| *Estimates were obtained from a mixed model for repeated measures, with *P. aeruginosa* status at baseline and maintenance use of macrolides at baseline as categorical fixed effects, interactions terms for treatment by visit and baseline by visit, and unstructured covariance matrix structure for repeated measurements within participants. | | | | |

### Patient-reported quality of life

With respect to the change from baseline in SGRQ Symptoms score at Week 24, the Compound (I) 2.5 mg group showed the largest numerical effect compared with placebo (Table 11). A similar trend was observed for SGRQ Total score. As there is no minimal clinically important difference for the SGRQ Symptoms score but only for the SGRQ Total score, the *post hoc* responder analysis was based on the SGRQ Total score. The percentage of responders (decrease from baseline in SGRQ Total score of ≥4 points) at Week 24 was greatest in the Compound (I) 2.5 mg group (62.7%), followed by the 5 mg (51.5%), placebo (47.6%) and 1 mg (45.1%) groups. At Week 48, the percentage of responders was still highest in the Compound (I) 2.5 mg group (75.8%) (Table 11, Figure 9).

The Compound (I) 1 mg dose showed the largest effect compared with placebo on change from baseline in QoL-B respiratory symptoms score at Week 24. *Post hoc* responder analysis showed that the Compound (I) 5 mg group had the greatest proportion of responders (change from baseline in QoL-B respiratory symptoms score of ≥8 points) at Week 24 (47.5%), followed by the 2.5 mg (45.1%), placebo (37.9%) and 1 mg (34.6%) groups. By Week 48, the Compound (I) 2.5 mg group (54.5%) had the greatest proportion of responders (Table **12,** Figure 10).

**Table 11: ABSOLUTE CHANGE FROM BASELINE IN SGRQ SYMPTOMS SCORE AND SGRQ TOTAL SCORE AT WEEK 24* AND POST HOC RESPONDER ANALYSIS† FOR SGRQ TOTAL SCORE AT WEEKS 24 AND 48.**

| | **Placebo** | **Compound (I)** | | | |
|---|---|---|---|---|---|
| | | **1 mg** | **2.5 mg** | **5 mg** | |
| **Week 24** | | | | | |
| *SGRQ Symptoms score* | | | | | |
| Absolute change from baseline versus placebo - adjusted mean (95% CI) | | - | -1.58 (-7.59 to 4.43) | -3.33 (-9.38 to 2.72) | -2.29 (-7.26 to 2.68) |
| *SGRQ Total score* | | | | | |
| Absolute change from baseline versus placebo - adjusted mean (95% CI) | | - | -1.23 (-5.74 to 3.28) | -3.03 (-7.54 to 1.48) | -2.57 (-6.28 to 1.14) |
| *SGRQ Total score post hoc* responder analysis | | | | | |
| | Number of participants | 103 | 51 | 51 | 99 |
| | Responders - n (%) | 49 (47.6) | 23 (45.1) | 32 (62.7) | 51 (51.5) |
| | Comparison versus placebo - difference of proportions (95% CI) | - | -2.5 (-18.5 to 14.0) | 15.2 (-1.5 to 30.4) | 3.9 (-9.7 to 17.4) |

| **Week 48** | | | | | |
|---|---|---|---|---|---|
| *SGRQ Total score* | | | | | |
| *SGRQ Total score post hoc* responder analysis | | | | | |
| | Number of participants | 65 | 34 | 33 | 63 |
| | Responders - n (%) | 31 (47.7) | 19 (55.9) | 25 (75.8) | 35 (55.6) |
| | Comparison versus placebo - difference of proportions (95% CI) | - | 8.2 (-12.1 to 27.4) | 28.1 (7.5 to 44.4) | 7.9 (-9.2 to 24.3) |

**Table 12: ABSOLUTE CHANGE FROM BASELINE IN QOL-B RESPIRATORY SYMPTOMS DOMAIN SCORE AT WEEK 24* AND POST HOC RESPONDER ANALYSIS† AT WEEKS 24 AND 48.**

| | **Placebo** | **Compound (I)** | | | |
|---|---|---|---|---|---|
| | | **1 mg** | **2.5 mg** | **5 mg** | |
| **Week 24** | | | | | |
| Absolute change from baseline versus placebo - adjusted mean (95% CI) | - | 2.16 (-2.75 to 7.06) | 1.72 (-3.22 to 6.65) | 0.95 (-3.11 to 5.01) | |

| *Post hoc* responder analysis | | | | | |
|---|---|---|---|---|---|
| | Number of participants | 103 | 52 | 51 | 99 |
| | Responders - n (%) | 39 (37.9) | 18 (34.6) | 23 (45.1) | 47 (47.5) |
| | Comparison versus placebo - difference of proportions (95% CI) | - | -3.2 (-18.2 to 12.9) | 7.2 (-8.8 to 23.3) | 9.6 (-4.0 to 22.7) |

| **Week 48** | | | | | |
|---|---|---|---|---|---|
| *Post hoc* responder analysis | | | | | |
| | Number of participants | 65 | 34 | 33 | 61 |
| | Responders - n (%) | 20 (30.8) | 14 (41.2) | 18 (54.5) | 25 (41.0) |
| | Comparison versus placebo - difference of proportions (95% CI) | - | 10.4 (-8.7 to 29.7) | 23.8 (3.3 to 42.3) | 10.2 (-6.4 to 26.2) |

### Safety

Adverse events (AEs) are summarised in Table 13. The incidence of AEs, including investigator-reported drug-related AEs, was similar across groups. Severe AEs were reported in 11.9% of the placebo group, and 9.4%, 7.5% and 15.0% of the Compound (I) 1 mg, 2.5 mg and 5 mg groups, respectively. AEs leading to treatment interruption were highest in the Compound (I) 5 mg group and AEs leading to treatment discontinuation were highest in the placebo group. No AESIs (potential severe DILI) were reported in any group. Mode of action-related AEs (skin, periodontal and opportunistic infection AEs) were infrequent, generally similar across groups, and mostly of mild or moderate intensity. Serious AEs were highest in the placebo group. Increased risk of infection was not seen with Compound (I).

**Table 13: SUMMARY OF ADVERSE EVENTS DURING TREATMENT**

| **Event*** | | | **Placebo (n=109)** | **Compound (I)** | | |
|---|---|---|---|---|---|---|
| | | | | **1 mg (n=53)** | **2.5 mg (n=53)** | **5 mg (n=107)** |
| Any adverse event | | | 101 (92.7) | 45 (84.9) | 46 (86.8) | 94 (87.9) |
| Any adverse event, excluding bronchiectasis exacerbations | | | 93 (85.3) | 43 (81.1) | 41 (77.4) | 82 (76.6) |

| Most frequent adverse events^{†} | | | | | | |
|---|---|---|---|---|---|---|
| | Bronchiectasis^{‡} | | 35 (32.1) | 18 (34.0) | 16 (30.2) | 35 (32.7) |
| | Infective exacerbation of bronchiectasis^{‡} | | 28 (25.7) | 11 (20.8) | 8 (15.1) | 18 (16.8) |
| | Nasopharyngitis | | 12 (11.0) | 5 (9.4) | 8 (15.1) | 11 (10.3) |
| | Cough | | 10 (9.2) | 2 (3.8) | 9 (17.0) | 10 (9.3) |
| | COVID-19 | | 12 (11.0) | 5 (9.4) | 6 (11.3) | 6 (5.6) |
| | Headache | | 11 (10.1) | 7 (13.2) | 2 (3.8) | 6 (5.6) |
| Serious adverse event | | | 30 (27.5) | 8 (15.1) | 6 (11.3) | 22 (20.6) |
| | Resulted in death | | 0 (0.0) | 0 (0.0) | 0 (0.0) | 1 (0.9)^{§} |
| Severe adverse event | | | 13 (11.9) | 5 (9.4) | 4 (7.5) | 16 (15.0) |
| Investigator-reported drug-related adverse event | | | 16 (14.7) | 8 (15.1) | 9 (17.0) | 18 (16.8) |
| Adverse event leading to treatment interruption | | | 10 (9.2) | 4 (7.5) | 2 (3.8) | 11 (10.3) |
| Adverse event leading to treatment discontinuation | | | 6 (5.5) | 2 (3.8) | 2 (3.8) | 5 (4.7) |
| Adverse event of special interest | | | 0 (0.0) | 0 (0.0) | 0 (0.0) | 0 (0.0) |

| Mode of action-related adverse event | | | | | | |
|---|---|---|---|---|---|---|
| | *Skin adverse event* | | 7 (6.4) | 2 (3.8) | 3 (5.7) | 12 (11.2) |
| | | Skin exfoliation | 1 (0.9) | 1 (1.9) | 1 (1.9) | 7 (6.5) |
| | | Hyperkeratosis | 2 (1.8) | 0 (0.0) | 2 (3.8) | 4 (3.7) |
| | | Erythema | 1 (0.9) | 0 (0.0) | 0 (0.0) | 4 (3.7) |
| | | Palmoplantar keratoderma | 2 (1.8) | 1 (1.9) | 0 (0.0) | 2 (1.9) |
| | | Palmar erythema | 1 (0.9) | 0 (0.0) | 0 (0.0) | 1 (0.9) |
| | | Plantar erythema | 0 (0.0) | 0 (0.0) | 0 (0.0) | 2 (1.9) |
| | *Periodontal adverse event* | | 5 (4.6) | 4 (7.5) | 1 (1.9) | 3 (3.8) |
| | | Noninfective gingivitis | 3 (2.8) | 1 (1.9) | 0 (0.0) | 2 (1.9) |
| | | Gingival recession | 1 (0.9) | 1 (1.9) | 1 (1.9) | 0 (0.0) |
| | | Periodontitis | 1 (0.9) | 1 (1.9) | 0 (0.0) | 1 (0.9) |
| | | Gingival disorder | 1 (0.9) | 0 (0.0) | 0 (0.0) | 0 (0.0) |
| | | Gingival erythema | 0 (0.0) | 0 (0.0) | 0 (0.0) | 1 (0.9) |
| | | Gingival pain | 0 (0.0) | 1 (1.9) | 0 (0.0) | 0 (0.0) |
| | | Gingival ulceration | 1 (0.9) | 0 (0.0) | 0 (0.0) | 0 (0.0) |
| | *Opportunistic infection adverse event* | | 1 (0.9) | 2 (3.8) | 0 (0.0) | 1 (0.9) |
| | | Stenotrophomonas infection | 0 (0.0) | 1 (1.9) | 0 (0.0) | 0 (0.0) |
| | | Aspergillus infection | 0 (0.0) | 1 (1.9) | 0 (0.0) | 0 (0.0) |
| | | Bronchopulmonary aspergillosis | 1 (0.9) | 0 (0.0) | 0 (0.0) | 0 (0.0) |
| | | Oesophageal candidiasis | 0 (0.0) | 0 (0.0) | 0 (0.0) | 1 (0.9) |

| | | | | | | |
|---|---|---|---|---|---|---|
| *Adverse events were classified using the Medical Dictionary for Drug Regulatory Activities v27. Data are presented as n (%). ^{†}The most frequent adverse events were defined as those with an incidence of more than 10% in at least one of the trial groups. ^{‡}Investigators coded pulmonary exacerbation adverse events as either 'bronchiectasis' or 'infective exacerbation of bronchiectasis'. As such, these terms are interchangeable. | | | | | | |

### NE activity in Sputum

Sputum NE activity was quantified to determine how target engagement in blood, demonstrated in healthy volunteer trials, translated into target engagement in patients' sputum as surrogate for the target organ lung.

Placebo corrected sputum NE inhibition up to Week 24 are provided in Table 14. Absolute change from baseline in log10-transformed NE activity, up to Week 24, are shown in Figure 14. A dose dependent reduction in NE activity was observed for Compound (I).

NE activity was measured in either spontaneous or induced sputum samples using the relative-quantitative assay. Baseline mean NE activity in sputum was similar across treatment groups. At week 12 NE inhibition (secondary endpoint) reached 48.2% in the 1 mg, 85.2% in 2.5 mg and 86.4% in the 5 mg dose groups. Compared to placebo the observed inhibition was 24.1%, 78.4% and 80.2% in the 1, 2.5 and 5 mg dose groups, respectively. The inhibition of NE activity was dose-dependent (Table 14, Figure 14) with the 5 mg dose showing highest inhibition of NE activity and reaching up to 91% inhibition. Compared to patients receiving 2.5 mg q.d., patients receiving the 5 mg q.d. showed a steeper decline in sputum NE activity within the first four weeks. For both doses, maximal levels of inhibition are observed after 8 weeks (Figure 14).

**Table 14: MEAN PLACEBO CORRECTED NE INHIBITION UP TO WEEK 24 PER TREATMENT GROUP (TREATED SET). BASED ON A MIXED MODEL FOR REPEATED MEASURES (MMRM) IN CHANGE FROM BASELINE IN NE ACTIVITY (LOG-10 TRANSFORMED).**

| **Dose Group** | **1 mg** | **2.5 mg** | **5 mg** |
|---|---|---|---|
| Week 4 | 8.7 % | 40.5% | 85.0% |
| Week 8 | 65.1% | 83.9% | 91.7% |
| Week 12 | 24.1% | 78.4% | 80.2% |
| Week 24 | 44.9% | 78.9% | 90.8% |

### CLAIRAFLY (1397-0013)

### Description of the trial:

This clinical trial was a multinational, randomised, double-blind, placebo-controlled, parallel group trial evaluating safety, tolerability, pharmacodynamics and pharmacokinetics of Compound (I) 5mg once daily.

A historical clinical diagnosis of CF and an investigator-confirmed diagnosis of bronchiectasis by CT scan are the main sources for the diagnosis for trial entry. Following a screening period of 6 weeks, participants were randomized in 2:1 to receive placebo or 5mg of Compound (I). for 12 weeks followed by a 4 weeks safety follow up.

The purpose of this study was to find out whether the Compound (I) is safe with similar pharmacokinetic and pharmacodynamic properties in patients with cystic fibrosis bronchiectasis (CFB) compared to patients with non-cystic fibrosis bronchiectasis (NCFB) investigated in AIRLEAF.

### STUDY POPULATION

A total of 22 participants were randomised and received Compound (I) 5 mg (n=15) or placebo (n=7). All patients completed the study. Seven (46.7%) patients on stable maintenance use of CFTR modulator treatment were treated with Compound (I) 5mg group and 4 (57.1%) were in placebo group.

### SAMPLE SIZE DETERMINATION

The planned sample size was not based on a power calculation but was considered sufficient for the exploratory evaluation of safety and tolerability of Compound (I) as well as to investigate PD and PK properties.

### TRIAL OBJECTIVES AND ENDPOINTS

The primary objective was to estimate the number and percentage of patients who had at least one TEAE during the trial. The secondary objective was to estimate the PD effect assessed by relative change from baseline in neutrophil elastase (NE) in sputum at Week 8, after the first drug administration. The other secondary objective was to estimate the PK effect assessed by the area under the concentration-time curve of the analyte in plasma over a dosing interval (AUCτ) for the first dose, the maximum concentration (Cmax) for the first dose, the AUCτ at steady state (AUCτ,ss) and the Cmax at steady state (Cmax,ss) after multiple dosing of Compound (I) 5 mg once daily.

### STATISTICAL ANALYSES

The primary endpoint and secondary endpoints were evaluated using descriptive statistics. All evaluations were to be considered exploratory. Safety and secondary PD analyses were based on the treated set (TS) which included all patients who were dispensed study medication and were documented to have taken at least one dose of investigational treatment. Analyses of PK parameters were based on the PK parameter analysis set which included those patients in the TS with at least one valid plasma concentration value available.

### RESULTS ON SAFETY, PK AND PD ENDPOINTS.

No relevant differences in demographics and baseline characteristics were observed between both treatment groups.

### Safety results

In total, the vast majority of patients reported any TEAE (6 patients, 85.7% in the placebo group, 14 patients, 93.3% in the Compound (I) 5 mg group). The proportions of patients who reported any TEAE were comparable between both treatment groups. The most frequently reported TEAE by preferred term (PT) was infective pulmonary exacerbation of cystic fibrosis, followed by headache. Overall, 6 patients (27.3%) experienced treatment-emergent SAEs (1 patient, 14.3% in the placebo group and 5 patients, 33.3% in the Compound (I) 5 mg group). Skin exfoliation was reported for 2 patients (13.3%) of the Compound (I) 5 mg group and gingival recession for 1 patient (6.7%) of the Compound (I) 5 mg group. No infection TEAEs were reported during this trial. No deaths, treatment-emergent AESIs which were prespecified as potential severe DILI, or other significant TEAEs were reported. All drug-related TEAEs were non-serious, of mild intensity, and were resolved by the end of the trial.

Overall, once daily doses of 5 mg Compound (I) were well tolerated by the patients in this trial.

### Pharmacokinetic results

### PK blood samples were collected for the determination of trough Compound (I)

plasma concentrations on trial Days 1 (Visit 2), 8 (Visit 3), 29 (Visit 4), 57 (Visit 5), and 85 (Visit 6, EOT) and were analysed descriptively.

Compound (I) plasma concentration-time profiles were similar after single and multiple oral administrations with median tmax (ss) around 3 h. The exposures at steady state were higher compared to exposures after single dose, indicating accumulation. Overall, the gMean trough concentrations remained consistent from Day 29 onwards, indicating that Compound (I) reached steady state by Day 29. The gMean trough concentrations at steady state were comparable regardless of CFTR modulator use.

In this trial, the steady state exposure of Compound (I) was comparable to that observed in the 1397-0012 Trial conducted in patients with non-cystic fibrosis bronchiectasis.

In summary, PK of Compound (I) in patients with non-cystic BE and in patients with cystic fibrosis BE is similar.

### Pharmacodynamic results

The median percent inhibition of NE activity in sputum at Week 8 was 89.4% (range: -60.2% to 100%) for the Compound (I) 5 mg group.

For the subgroup of patients on stable maintenance use of CFTR-MT at baseline, the median percent inhibition of NE activity in sputum at Week 8 was 94.2% (range: -6.4% to 100%) in

the Compound (I) 5 mg group. For the subgroup of patients not on stable maintenance use of CFTR-MT at baseline, the median percent inhibition of NE activity in sputum at Week 8 was 73.7% (range: -60.2% to 97.8%) in the Compound (I) 5 mg group. The median NE inhibition at all timepoints is provided in Table 15.

A comparison of sputum NE across AIRLEAF and CLAIRAFLY is shown in Figure 15. In summary, NE inhibition by Compound (I) in patients with non-cystic fibrosis BE and in patients with cystic fibrosis BE is in the same range.

**Table 15 MEDIAN NE INHIBITION UP TO WEEK 12 BY STABLE TREATMENT WITH CFTR MODULATOR (TREATED SET).**

| | **Overall population** | **CFTR-MT: YES** | **CFTR-MT: NO** |
|---|---|---|---|
| **Dose Group** | **Compound (I) 5 mg** | **Compound (I) 5 mg** | **Compound (I) 5 mg** |
| Week 4 | 88.6% | 96.8% | 84.3% |
| Week 8 | 89.4% | 94.2% | 73.7% |
| Week 12 | 89.0% | 98.0% | 67.4% |

In an in vitro NETosis assay the effect of Compound (I) on the formation of neutrophil extracellular traps (NETs) was evaluated. As a result, Compound (I) can significantly decrease NET formation by neutrophils derived from human CD34+ progenitor cells.

### IN VITRO NETOSIS ASSAY

### Materials, reagents and equipment

Compound (I) Human Cord Blood CD34+ Stem/Progenitor Cells, Cryopreserved, 1 million (Lonza, Cat # 2C-101)
StemSpan SFEM II media (Stemcell technologies, Cat# 09605)
StemSpan^{™} CD34+ Expansion Supplement (10X) (Stemcell technologies, Cat # 02691) RPMI 1640 Medium (Gibco, Cat# 11875093)
Fetal Bovine Serum, qualified, heat inactivated, United States (Gibco, Cat# 16140071) Penicillin-Streptomycin (10,000 U/mL) (Gibco, Cat# 15140122)
Recombinant G-CSF (Peprotech, Cat# 300-23-10UG)
DMSO (Sigma, Cat# D2650)
Macrophage-SFM (1X) (Gibco, Cat# 12065074)
PMA (Sigma, Cat# P1585)
YOYO-1 Iodide (Dimeric Cyanine Nucleic Acid Stains) (Invitrogen, Cat# Y3601) N-Formyl-Met-Leu-Phe (fMLP) (Sigma, Cat# 47729)
Tissue culture 24 well plate (Corning, 3527)
Tissue culture 96 well plate (ThermoFisher, Cat# 167542)

### Media preparation

CD34+ expansion media: 9mL of SFEM II media supplemented with 1mL of 10X CD34+ expansion supplement.

Neutrophil differentiation media: RPMI1640 supplemented with 10% FBS, 10ng/ml G-CSF, and 100 U/mL Penicillin-Streptomycin.

### Compound preparation

1 mg of Compound (I) is dissolved in 188.5µL of 100% DMSO at a concentration of 10mM. Compound (I) is then diluted to 2mM and 0.4mM in 100% DMSO and then secondarily diluted to 50µM, 10 µM, 2 µM, and 0.4µM in neutrophil differentiation media, giving rise to 10X solutions for treatment.

PMA is dissolved in 100% DMSO at concentration of 1.6mM. 1.6mM PMA is further diluted to 16uM in 100% DMSO and then secondarily diluted to 1µM in Macrophage-SFM and then further diluted 10nM, making 10X stock solution.

10mg of fMLP is dissolved in 333µL of 100% DMSO at a concentration of 68.6mM. 68.6mM fMLP is then diluted to 16mM in 100% DMSO. 16mM fMLP is further diluted to 1mM in Macrophage-SFM and then secondarily diluted to 10µM in Macrophage-SFM, generating a 10X solution for treatment.

YOYO-1 Iodide is diluted to 0.2µM in macrophage-SFM from 1mM solution, generating a 10X solution for treatment.

### CatC inhibition with Compound (I) during neutrophil differentiation

Human cord blood derived CD34+ progenitor cells are thawed into CD34+ expansion media at the density of 1x10⁶ cells/mL in a 24-well plate. Cells are incubated at 37°C, 5% CO₂ for 24 hours for recovery. On the next day, cells are resuspended at 0.56 x 10⁶ cells/mL in neutrophil differentiation media and divided into 6 wells on a 24-well plate (e.g., 0.3 x 10⁶ cells in 540µL per well or 0.2 x 10⁶ cells in 360 µL per well). Compound (I) is then added into each well at final concentrations of 0.04uM, 0.2uM, and 1uM. This is considered as day 0 of the differentiation protocol and media change is performed every 48 hours for 10 days. At each media change, cells are recounted and resuspended at the density of 1 x 10⁶ cells/mL in neutrophil differentiation media followed by a retreatment of Compound (I) at 0.04uM, 0.2uM, and 1uM. On day 10, differentiated cells are collected and used for NETosis assay.

### In vitro NETosis assay

Differentiated neutrophils are counted and resuspended in macrophage-SFM and seeded at 1.5x10⁴ cells per well in a 96-well plate. The cells are then treated with PMA (1nM) or fMLP (1uM) to induce NETosis. A fluorescence DNA dye, YOYO-1 Iodide, is also added to the treatment at 0.2 uM to allow visualization of extracellular DNA. NET formation is monitored via live-cell imaging using Incucyte-SX5 analysis system. Images are taken every 2 hours for 24 hours. The intensity threshold for NETosis detection is set at minimal of 2.0 GCU and the minimal NET size is set at 400 µm².

### Data analysis

Fluorescence intensity is multiplied by total imaging area (GCU*µm²) to generate Integrated Intensity at each time point using the Incucyte 2023 or Incucyte 2022A software. AUC is generated in Graphpad Prism 10.1.2 by plotting Integrated Intensity values between 1 to 16 hours for PMA treatment or 1 to 22 hours for fMLP treatment. Percentage of AUC to PMA or fMLP treatment is generated using the following formula: percentage AUC= (AUC_{X}/AUC_{PMA}) * 100 or percentage AUC = (AUC_{X}/AUC_{fMLP}) * 100 _{;} x = indicated treatment condition.

### Results

Human cord blood-derived CD34+ progenitor cells were treated with 1µM, 0.2µM or 0.04µM of Compound (I) or with DMSO during in vitro neutrophils differentiation for 10 days. On day 10, differentiated cells were stimulated with PMA at 1nM or with fMLP at 1uM to induce NETosis. Difference in NETosis induction between each condition is presented as percentage of AUC normalized to the AUC of PMA- or fMLP-alone condition. Compared to DMSO control, CatC inhibition with 1uM Compound (I) during differentiation led to an over 90% reduction in NETosis induced by PMA over 16 hours. Additionally, NETosis in cells differentiated under 0.2 µM or 0.04 µM of Compound (I) was 85% less following PMA treatment compared to cells differentiated under DMSO (Figure 1N). Since PMA initiates NETosis via PKC activation, we next investigated whether CatC inhibition can block NETosis induced by fMLP, which activates NETosis pathway in a PKC-independent manner. Following fMLP treatment, there was an 85% reduction in NETosis in cells differentiated under 1µM Compound (I) compared to DMSO control over 22 hours (Figure 2N). In summary, these data suggest that inhibition of CatC by Compound (I) can block NETosis induced by PMA or fMLP.

Since neutrophil activation resulting in NETosis and NSP release are key pathogenic drivers of recurring neutrophilic inflammation in HS and ANCA-AV, treatment with Compound (I) is expected to result in a clinically meaningful reduction of HS and ANCA-AV disease severity.

### PREPARATION OF POLYMORPH FORM [III] AND FORM [I].

Compound (I) is prepared as described in Vintonyak et al WO2016038007A1, Example 1: see, in particular, the methodology starting from page 37, line 9 "Method A" to page 46, line 13 and said methodology section is incorporated herein. There is no disclosure in Vintonyak *et al* on the formation of a crystalline CatC inhibitor. Step 6 on page 46 of Vintonyak *et al* discloses purification by chromatography and the standard isolation procedure after such HPLC is freeze drying, resulting in amorphous material.

### Crystallization method for preparation of Form [III]

Compound (I), as described in Vintonyak et al WO2016038007A1, obtained in preparation step 6 of Example 1 therein, (1.0 g) is suspended in n-butyl acetate (nBuOAc) (16.5 mL) at 20 °C. The slurry is heated to 55-60 °C for dissolution, filtered at ~60 °C, and rinsed with nBuOAc (1 mL). The batch is then seeded with Compound (I) (1.0 mg) and aged for ~1 hour. The batch is then distilled under vacuum (4.3-3.6 psi) to remove the solvent level (12.9 mL) and then cooled down to 50 °C. Then the batch charges methyl-tert-butyl ether (MTBE) as antisolvent for 8 mL and slurry for 2 hrs. Keep cooling down the batch to 5 °C over 2 hours, the batch is filtered and washed with MTBE (2.0 mL). The product is dried in an oven at about 60 °C under reduced pressure for not less than 8 hours.

Seed crystals of Form [III] can be obtained by crystallization of Compound of formula (I) in n-butyl acetate (nBuOAc).

### Crystallization method for the preparation of Form [I]

Form [III] (1.0 g) is suspended in ethanol (5.0 mL) at 20 °C and the slurry is kept over 8 hours. The batch is filtrated and washed with ethanol (1.0 mL) and dried at room temperature, e.g. 20 °C, overnight to get Form [I] (ethanol solvate).

X-RAY POWDER DIFFRACTION DIAGRAM (XRPD) ANALYSIS OF FORM [III] AND FORM [I].

The polymorphs/crystalline forms of the compound of formula (I) were characterized by X-ray powder diffraction (XRPD) as shown in Figures 1P and 2P showing the X-ray powder diffraction diagrams for each of Form [III] and Form [I].

The characteristic peak positions and relative intensities for the XRPD pattern in Figure 1P and Figure 2P is shown in Table 1P to Table 6P below.

For the performance of the X-ray powder diffraction analysis a Bruker D8 ADVANCE instrument was used with an X-ray generator of the type Power 1600 W (40 kV-40 mA) (Optics: motorized divergence slit). The Goniometer range was 2.0 - 35.0 ° 2 θ and the scan speed was 0.015° 2 θ/step with an accuracy of 0.5 sec/step. A LynxEye position sensitive detector was used. The sample was analyzed on a low background Si (510) sample holder.

### XRPD analysis of Form [III]

The XRPD pattern of Form [III] is shown in Figure 1P. The characteristic peak positions and relative intensities for the XRPD pattern in Figure 1P are shown in Table 1P-(2θ -Theta-values and d-values) below.

**Table 1P: All observable peaks of Form [III] (see Figure 1P):**

| **2-Theta (degrees)** | **d(Å)** | **I/Io** |
|---|---|---|
| 6.2 | 14.33 | 0.47 |
| 6.9 | 12.74 | 0.02 |
| 7.2 | 12.26 | 0.28 |
| 11.5 | 7.70 | 0.08 |
| 12.3 | 7.16 | 0.31 |
| 12.8 | 6.93 | 0.06 |
| 13.9 | 6.37 | 0.10 |
| 14.2 | 6.25 | 0.05 |
| 14.5 | 6.12 | 0.10 |
| 14.6 | 6.04 | 0.15 |
| 16.8 | 5.26 | 0.59 |
| 17.2 | 5.16 | 0.67 |
| 17.4 | 5.11 | 0.57 |
| 17.8 | 4.99 | 1.00 |
| 18.1 | 4.90 | 0.87 |
| 18.2 | 4.87 | 0.69 |
| 18.6 | 4.78 | 0.07 |
| 18.9 | 4.69 | 0.10 |
| 19.3 | 4.60 | 0.29 |
| 19.6 | 4.53 | 0.47 |
| 20.8 | 4.26 | 0.06 |
| 21.3 | 4.17 | 0.32 |
| 21.6 | 4.12 | 0.29 |
| 22.1 | 4.02 | 0.29 |
| 22.3 | 3.98 | 0.31 |
| 22.6 | 3.94 | 0.07 |
| 22.9 | 3.88 | 0.28 |
| 23.1 | 3.85 | 0.26 |
| 23.4 | 3.80 | 0.04 |
| 24.6 | 3.62 | 0.07 |
| 24.8 | 3.58 | 0.02 |
| 25.3 | 3.51 | 0.14 |
| 25.6 | 3.48 | 0.05 |
| 25.8 | 3.45 | 0.05 |
| 26.3 | 3.38 | 0.19 |
| 26.4 | 3.37 | 0.14 |
| 26.7 | 3.33 | 0.10 |
| 27.4 | 3.25 | 0.07 |
| 28.0 | 3.18 | 0.07 |
| 28.5 | 3.13 | 0.04 |
| 28.5 | 3.12 | 0.05 |
| 28.8 | 3.10 | 0.02 |
| 29.1 | 3.06 | 0.07 |
| 29.8 | 2.99 | 0.04 |
| 30.6 | 2.92 | 0.04 |
| 30.9 | 2.89 | 0.03 |
| 31.2 | 2.86 | 0.06 |
| 31.7 | 2.82 | 0.02 |
| 31.9 | 2.81 | 0.03 |
| 32.3 | 2.77 | 0.04 |
| 32.5 | 2.75 | 0.05 |
| 33.2 | 2.69 | 0.03 |
| 33.3 | 2.69 | 0.02 |
| 33.8 | 2.65 | 0.04 |
| 34.1 | 2.63 | 0.01 |

The major peaks of the XRPD diagram of Form [III] are listed in Table 2P (see Figure 1P).

**Table 2P: Major peaks of Form [III]**

| **2-Theta (degrees)** | **d(Å)** | **I/Io** |
|---|---|---|
| 17.8 | 4.99 | 1.00 |
| 18.1 | 4.90 | 0.87 |
| 18.2 | 4.87 | 0.69 |
| 17.2 | 5.16 | 0.67 |
| 16.8 | 5.26 | 0.59 |
| 17.4 | 5.11 | 0.57 |
| 6.2 | 14.33 | 0.47 |
| 19.6 | 4.53 | 0.47 |
| 21.3 | 4.17 | 0.32 |
| 12.3 | 7.16 | 0.31 |
| 19.3 | 4.60 | 0.29 |
| 22.1 | 4.02 | 0.29 |
| 21.6 | 4.12 | 0.29 |
| 7.2 | 12.26 | 0.28 |

The most prominent peaks of the XRPD diagram of Form [III] are listed in Table 3P (see Figure 1P).

**Table 3P: Prominent peaks of Form [III]**

| **2-Theta (degrees)** | **d(Å)** | **I/Io** |
|---|---|---|
| 6.2 | 14.33 | 0.47 |
| 7.2 | 12.26 | 0.28 |
| 12.3 | 7.16 | 0.31 |
| 17.8 | 4.99 | 1.00 |
| 18.1 | 4.90 | 0.87 |

### XRPD analysis of Form [I]

The XRPD pattern of Form [I] of the compound of formula (I) is shown in Figure 2P. The characteristic peak positions and relative intensities for the XRPD pattern in Figure 2P are shown in Table 4P-(2θ -Theta-values and d-values) below.

**Table 4P: All observable peaks of Form [I] (Figure 2P)**

| **2-Theta (degrees)** | **d(Å)** | **I/Io** |
|---|---|---|
| 8.0 | 11.07 | 0.61 |
| 9.3 | 9.46 | 0.17 |
| 10.5 | 8.45 | 0.18 |
| 11.5 | 7.66 | 0.34 |
| 13.5 | 6.53 | 0.31 |
| 13.8 | 6.43 | 0.07 |
| 14.1 | 6.26 | 0.31 |
| 14.7 | 6.04 | 0.04 |
| 16.0 | 5.54 | 0.35 |
| 16.2 | 5.48 | 0.14 |
| 16.5 | 5.38 | 0.16 |
| 16.9 | 5.24 | 0.75 |
| 17.4 | 5.10 | 0.90 |
| 18.1 | 4.90 | 0.82 |
| 18.7 | 4.74 | 0.15 |
| 19.3 | 4.59 | 1.00 |
| 20.0 | 4.44 | 0.26 |
| 21.0 | 4.22 | 0.23 |
| 21.9 | 4.06 | 0.42 |
| 22.1 | 4.03 | 0.25 |
| 22.7 | 3.92 | 0.35 |
| 23.5 | 3.78 | 0.06 |
| 24.1 | 3.69 | 0.02 |
| 24.3 | 3.66 | 0.04 |
| 24.9 | 3.58 | 0.09 |
| 25.2 | 3.54 | 0.06 |
| 25.7 | 3.46 | 0.62 |
| 26.5 | 3.36 | 0.08 |
| 26.6 | 3.35 | 0.08 |
| 27.1 | 3.29 | 0.22 |
| 27.8 | 3.21 | 0.05 |
| 28.2 | 3.16 | 0.02 |
| 28.5 | 3.13 | 0.02 |
| 29.0 | 3.08 | 0.13 |
| 29.6 | 3.01 | 0.06 |
| 30.1 | 2.97 | 0.05 |
| 30.3 | 2.95 | 0.02 |
| 31.1 | 2.88 | 0.02 |
| 31.4 | 2.85 | 0.02 |
| 32.3 | 2.77 | 0.03 |
| 32.6 | 2.74 | 0.04 |
| 32.9 | 2.72 | 0.04 |
| 33.4 | 2.68 | 0.02 |
| 33.8 | 2.65 | 0.02 |

The major peaks of the XRPD diagram of Form [I] are listed in Table 5P (see Figure 2P).

**Table 5P: Major peaks of Form [I]**

| **2-Theta (degrees)** | **d(Å)** | **I/Io** |
|---|---|---|
| 19.3 | 4.59 | 1.00 |
| 17.4 | 5.10 | 0.90 |
| 18.1 | 4.90 | 0.82 |
| 16.9 | 5.24 | 0.75 |
| 25.7 | 3.46 | 0.62 |
| 8.0 | 11.07 | 0.61 |
| 21.9 | 4.06 | 0.42 |
| 22.7 | 3.92 | 0.35 |
| 16.0 | 5.54 | 0.35 |
| 11.5 | 7.66 | 0.34 |
| 14.1 | 6.26 | 0.31 |
| 13.5 | 6.53 | 0.31 |
| 20.0 | 4.44 | 0.26 |
| 22.1 | 4.03 | 0.25 |
| 21.0 | 4.22 | 0.23 |
| 27.1 | 3.29 | 0.22 |
| 10.5 | 8.45 | 0.18 |
| 9.3 | 9.46 | 0.17 |

The most prominent peaks of the XRPD diagram of Form [I] are listed in Table 6P (see Figure 2P).

**Table 6P: Prominent peaks of Form [I]**

| **2-Theta (degrees)** | **d(Å)** | **I/Io** |
|---|---|---|
| 8.0 | 11.07 | 0.61 |
| 9.3 | 9.46 | 0.17 |
| 10.5 | 8.45 | 0.18 |
| 11.5 | 7.66 | 0.34 |
| 17.4 | 5.10 | 0.90 |
| 19.3 | 4.59 | 1.00 |

### THERMOGRAVIMETRIC ANALYSIS (TGA) OF FORM [III] AND FORM [I].

Crystalline Forms A and Form [I] are further characterized by thermogravimetric analysis (TGA). TA Instruments TGA Q5500 was used for TGA analysis. Approximately 10 mg of sample was placed on a tared platinum TGA pan. Sample was scanned from 25°C to 300°C with 10°C/min heating rate. TGA furnace was purged with 50ml/min nitrogen while the balance was purged with 40ml/min nitrogen (see Figure 3P and Figure 4P).

DIFFERENTIAL SCANNING CALORIMETRY (DSC) ANALYSIS OF THE FORM [III] AND FORM [I].

Crystalline Forms A and Form [I] are further characterized by thermogravimetric analysis (TGA).

TA Instruments DSC Q2500 was used for DSC Analysis. The sample was analyzed in an unsealed Aluminium pan under an N₂ flow. The ramp that was used for the measurement was 10°C/min from 20°C to 300°C.

DSC analysis of Form [III] indicates an endotherm at about 183.7 °C (see Figure 5P).

DSC analysis of Form [I] indicates a major endotherm at about 181.8 °C, and two endotherms about 119.2 °C and 137.1 °C (see Figure 6P).

### SOLID-STATE NMR:

Crystalline Forms A and Form [I] are further characterized by ¹⁹F and ¹³C solid-state NMR

### Solid-State ¹⁹F and ¹³C NMR spectrum of Form [III]

¹⁹F Solid-state NMR (SSNMR) data for a sample of crystalline Form [III] were acquired on a Bruker Avance NEO NMR spectrometer (Bruker Biospin, Inc., Billerica, MA) with a 9.4 T magnet (¹H=400.46 MHz, ¹⁹F= 376.76 MHz). Sample was packed in 3.2 mm O.D. zirconia rotors with Vespel^{®} drive tips. A Bruker model 3.2BL BB probe was used for data acquisition and sample spinning about the magic-angle (54.74 degrees). Sample spectra were acquired with a spinning rate of 22 kHz with variable temperature control adjusted so that the internal sample temperature was situated at ambient temperature and pressure. A standard pulse-echo sequence was used with a recycle delay of 60 sec. SWf-TPPM 1H decoupling was also employed. No exponential line broadening was used prior to Fourier transformation of the free induction decay. Chemical shifts are referenced using the most intense signal from polyvinylidene fluoride (PVDF), with the resonance set to -91.0 ppm. The magic-angle was set using the ⁷⁹Br signal from KBr powder at a spinning rate of 5 kHz. Exemplary ¹⁹F SSNMR spectra of the samples, and corresponding chemical shifts are found in Figure 7P and Table 7P, respectively. The values reported in Table 7P have an uncertainty of ± 0.5 ppm.

¹³C SSNMR data for a sample of crystalline Form [III] were acquired on a Bruker Avance III HD spectrometer (Bruker Biospin, Inc., Billerica, MA) with an 11.7 T magnet (¹H=500.28 MHz, ¹³C=125.79 MHz). A sample was packed in a 4.0 mm O.D. zirconia rotor with KEL-F^{®} drive tips. A Bruker model 500SB BL4 BB probe was used for data acquisition and sample spinning about the magic-angle (54.74 degrees) with a spinning rate of 12.5 kHz with variable temperature control adjusted so that the internal sample temperature was situated at ambient temperature and pressure. A standard cross-polarization pulse sequence was used with a ramped Hartman-Hahn match pulse on the proton channel. The pulse sequence uses an 8 millisecond contact pulse for crystalline Form [III] and PASS (phase-adjusted spinning sidebands) was used to achieve TOSS (total suppression of spinning sidebands). A 243 second recycle delay was used for Form [III] Swept-frequency two-pulse phase modulation (SWf-TPPM) 1H decoupling during acquisition was also employed in the pulse sequence. No exponential line broadening was used prior to Fourier transformation of the free induction decay. Chemical shifts are referenced using the secondary standard of adamantane, with the high frequency resonance being set to 38.48 ppm. The magic-angle was set using the ⁷⁹Br signal from KBr powder at a spinning rate of 5 kHz. An exemplary ¹³C SSNMR spectrum of the sample is found in Figure 8P, Table 8P includes the chemical shifts obtained from the ¹³C SSNMR spectrum, which have an uncertainty of ± 0.3 ppm.

The ¹⁹F and ¹³C Solid-State NMR spectrum of Form [III] is shown in Figure 7P and Figure 8P. The characteristic ¹⁹F and ¹³C chemical shifts of Form [III] are shown in Table 7P and Table 8P below.

**Table 7P: ¹⁹F chemical shifts of Form [III]**

| Peak | Chemical Shift (ppm) |
|---|---|
| 1* | -112.5 |
| 2 | -116.1 |
| 3* | -116.5 |

| | |
|---|---|
| * = peak intensity is dependent on temperature-induced ring flipping. | |

**Table 8P: ¹³C chemical shifts of Form [III]**

| Peak | Chemical Shift (ppm) |
|---|---|
| 1 | 174.0 |
| 2* | 172.4 |
| 3 | 161.9 |
| 4* | 161.0 |
| 5 | 160.0 |
| 6 | 149.2 |
| 7 | 143.0 |
| 8 | 140.5 |
| 9 | 138.4 |
| 10 | 134.4 |
| 11* | 130.5 |
| 12 | 125.5 |
| 13 | 123.6 |
| 14 | 122.2 |
| 15 | 120.3 |
| 16 | 119.3 |
| 17* | 115.4 |
| 18 | 113.9 |
| 19 | 85.2 |
| 20 | 78.3 |
| 21 | 76.5 |
| 22 | 71.2 |
| 23 | 64.1 |
| 24 | 58.4 |
| 25 | 57.0 |
| 26 | 47.3 |
| 27 | 42.3 |
| 28 | 38.4 |
| 29 | 36.6 |
| 30 | 35.4 |
| 31 | 30.6 |
| 32* | 29.7 |
| 33 | 27.6 |

| | |
|---|---|
| * = peak intensity is dependent on temperature-induced ring flipping. | |

### Solid-State 19F and 13C NMR spectrum of Form [I]

¹⁹F SSNMR data for a sample of crystalline Form [I] were acquired on a Bruker Avance NEO NMR spectrometer (Bruker Biospin, Inc., Billerica, MA) with a 9.4 T magnet (¹H=400.46 MHz, ¹⁹F= 376.76 MHz). Sample was packed in 3.2 mm O.D. zirconia rotors with Vespel^{®} drive tips. A Bruker model 3.2BL BB probe was used for data acquisition and sample spinning about the magic-angle (54.74 degrees). Sample spectra were acquired with a spinning rate of 22 kHz with variable temperature control adjusted so that the internal sample temperature was situated at ambient temperature and pressure. A standard pulse-echo sequence was used with a recycle delay of 30 sec. SWf-TPPM ¹H decoupling was also employed. No exponential line broadening was used prior to Fourier transformation of the free induction decay. Chemical shifts are referenced using the most intense signal from polyvinylidene fluoride (PVDF), with the resonance set to -91.0 ppm. The magic-angle was set using the ⁷⁹Br signal from KBr powder at a spinning rate of 5 kHz. Exemplary ¹⁹F SSNMR spectra of the samples, and corresponding chemical shifts are found in Figure 9P and Table 9P, respectively. The values reported in Table 9P have an uncertainty of ± 0.5 ppm.

¹³C Solid-state NMR (SSNMR) data for a sample of crystalline Form [I] were acquired on a Bruker Avance NEO spectrometer (Bruker Biospin, Inc., Billerica, MA) with an 9.4 T magnet (¹H=400.46 MHz, ¹³C=100.70 MHz). A sample was packed in a 3.2 mm O.D. zirconia rotor with VESPEL^{®} drive tips. A Bruker model 3.2BL BB probe was used for data acquisition and sample spinning about the magic-angle (54.74 degrees) with a spinning rate of 18 kHz with variable temperature control adjusted so that the internal sample temperature was situated at ambient temperature and pressure. A standard cross-polarization pulse sequence was used with a ramped Hartman-Hahn match pulse on the proton channel. The pulse sequence uses an 11 millisecond contact pulse for Form [I]. A 14.85 second recycle delay was used for Form [I]. Swept-frequency two-pulse phase modulation (SWf-TPPM) ¹H decoupling during acquisition was also employed in the pulse sequence. No exponential line broadening was used prior to Fourier transformation of the free induction decay. Chemical shifts are referenced using the secondary standard of adamantane, with the high frequency resonance being set to 38.48 ppm. The magic-angle was set using the ⁷⁹Br signal from KBr powder at a spinning rate of 5 kHz. An exemplary ¹³C SSNMR spectrum of the sample is found in Figure 10P. Table 10P includes the chemical shifts obtained from the ¹³C SSNMR spectrum, which have an uncertainty of ± 0.3 ppm.

The ¹⁹F and ¹³C Solid-State NMR spectrum of Form [I] of the compound of formula (I) is shown in Figure 9P and Figure 10P. The characteristic ¹⁹F and ¹³C chemical shifts of Form [I] are shown in Table 9P and Table 10P below.

**TABLE 9P: ¹⁹F chemical shifts of Form [I]**

| Peak | Chemical Shift (ppm) |
|---|---|
| 1 | -110.0 |

**TABLE 10P: ¹³C chemical shifts of Form [I]**

| Peak | Chemical Shift (ppm) |
|---|---|
| 1 | 173.7 |
| 2 | 164.6 |
| 3 | 163.9 |
| 4 | 162.0 |
| 5 | 148.2 |
| 6 | 142.5 |
| 7 | 141.8 |
| 8 | 135.9 |
| 9 | 135.1 |
| 10 | 127.2 |
| 11 | 125.1 |
| 12 | 123.8 |
| 13 | 120.8 |
| 14 | 118.6 |
| 15 | 117.0 |
| 16 | 112.8 |
| 17 | 84.3 |
| 18 | 75.0 |
| 19 | 74.4 |
| 20 | 70.2 |
| 21 | 62.8 |
| 22 | 59.0 |
| 23 | 56.6 |
| 24 | 56.3 |
| 25 | 47.2 |
| 26 | 41.7 |
| 27 | 40.7 |
| 28 | 40.2 |
| 29 | 38.7 |
| 30 | 34.5 |
| 31 | 32.6 |
| 32 | 29.7 |
| 33 | 21.3 |

### SOLUBILITY OF FORM [III]

Based on a Biopharmaceutics Classification System, a drug substance is considered highly soluble when the highest strength is soluble in 250 mL or less of aqueous media within the pH range of 1 to 6.8 at 37 ± 1°C.

The lowest solubility of crystalline Form [III] at physiological pH is 0.3 mg/ml at pH 6.8, therefore 75 mg of Form [III] is considered the highest dose that is soluble in 250 mL fluid (at pH 6.8). Consequently, crystalline Form [III] is considered highly soluble compound of BCS class 1. The solubility results are listed in Table 12P.

### Methods

### Solubility Evaluation:

Solubility studies are performed by equilibrating an excess amount of solid (8-30 mg/mL) in solutions in a variety of media (Table 12P) in amber vials on end-over-end rotator. Aqueous samples of varied pH were incubated at 37°C for 24 hr. At the end of the equilibration time, prior to dilution for HPLC analysis, samples were filtered using NANOSEP 0.2 µm PVDF filter/centrifuge tubes and directly diluted as necessary into an organic diluent prior to HPLC analysis. In aqueous solubility studies 0.02 M (0.15 I.S.) buffers were used.

### HPLC Method:

A crystalline Form [III] HPLC Method AP-21091 v.1.0 was used in this solubility study. Separation was achieved on X Bridge C8, 3.0 mm x 100 mm (3 µm) column heated to 50°C using an Agilent 1260 equipped with auto-sampler and diode array detector. The detection wavelength was 258 nm with bandwidth 8 nm and the flow rate was set to 0.6 mL/min. The total run time was 45 minutes. The injection volume used was 4 µL. A gradient method with conditions listed in Table 11P was used. The mobile phase A is composed of 0.2% potassium phosphate buffer pH 2.5 and mobile phase B is methanol. An acetonitrile/ 0.2% potassium phosphate buffer pH 2.5 (50:50) was used as the diluent.

**Table 11P:**

| HPLC gradient method. Mobile Phase A (0.2% Potassium Phosphate Buffer, pH 2.5) (%) | Mobile Phase B (Methanol) (%) | Time (minutes) |
|---|---|---|
| 85.0 | 15.0 | 0.0 |
| 80.0 | 20.0 | 5.0 |
| 72.0 | 28.0 | 9.0 |
| 55.0 | 45.0 | 26.0 |
| 30.0 | 70.0 | 30.0 |
| 20.0 | 80.0 | 35.0 |
| 20.0 | 80.0 | 45.0 |

**Table 12P:**

| Equilibrium solubility results **Conditions** | **Buffer** | **Solubility (mg/ml)** |
|---|---|---|
| 37°C @ 24 hr | HCl (pH 1.2) | 25.5 ± 0.082 |
| 37°C @ 24 hr | Na acetate buffer w. NaCl (pH 4.5) | 4.5 ± 0.069 |
| 37°C @ 24 hr | Na phosphate buffer w. NaCl (pH 6.8) | 0.3 ± 1.012 |
| 37°C @ 24 hr | FaSSIF (pH 6.5) | 1.1 ± 0.052 |
| 37°C @ 24 hr | FeSSIF (pH 5.0) | 18.8 ± 0.073 |
| 37°C @ 24 hr | SGF (pH 1.2) | 23.2 ± 0.016 |

| | | |
|---|---|---|
| KEY: FaSSIF/FeSSIF = fasted and fed state simulated intestinal fluids; SGF = simulated gastric fluid. | | |

The above results obtained with the Form [III] are unexpected because it is generally not possible to predict such solubility of the thermodynamically stable Form [III].

### PHARMACEUTICAL COMPOSITION

Compound (I) Form [III] and Form [I] can be administered as tablets or filmcoated tablets, as exemplified for Form [III] in the following manufacturing steps and in Table 13P1 and Table 13P2.

### Manufacturing of main blend (step 1):

Mannitol, Compound (I) Form [III], hydroxypropylcellulose, crospovidone and microcrystalline cellulose are blended in a suitable diffusion mixer followed by a screening step.

The screened mixture is blended in a suitable diffusion mixer to obtain the main blend.

### Manufacturing of final blend (step 2):

The main blend is screened together with magnesium stearate.

The mixture is subsequently blended in a suitable diffusion blender to obtain the final blend. Manufacturing of tablets (step 3).

The final blend is compressed into tablet cores using a suitable rotary tablet press.

### Film coating method (step 4):

The film-coating mixture is dispersed in purified water by stirring in a suitable mixing vessel to obtain the film-coating suspension.

The tablet cores are coated with the film-coating suspension using a suitable pan coater. As an alternative to calcium carbonate, TiO₂ may be used.

**TABLE 13P1 QUALITATIVE AND QUANTITATIVE COMPOSITION OF COMPOUND (I) FILM-COATED TABLETS, 2.5 MG AND 5 MG**

| **Ingredients** | **2.5 mg** | **5 mg** | **%/tablet** | **Function** | **Reference to standards** |
|---|---|---|---|---|---|
| | **[mg/tablet]** | **[mg/tablet]** | | | |
| Compound (I) | 2.50 | 5.00 | 3.13 | Active ingredient | Company standard |
| Cellulose, microcrystalline | 24.50 | 49.00 | 30.63 | Filler | Ph.Eur./NF |
| Mannitol | 49.00 | 98.00 | 61.25 | Filler | Ph.Eur./USP |
| Hydroxypropylcellulose | 2.40 | 4.80 | 3.00 | Binder | Ph.Eur./NF |
| Crospovidone | 0.30 | 0.60 | 0.38 | Disintegrant | Ph.Eur./NF |
| Magnesium stearate | 1.30 | 2.60 | 1.63 | Lubricant | Ph.Eur./NF |
| **Total (tablet)** | **80.0** | **160.0** | **100.0** | | |
| Film-coating mixture, yellow | 5.00 ⁶ | 9.00 ⁷ | - | Film-coating agent | Company standard |
| Water, purified | *(28.33)* | *(51.00)* | - | Solvent | Ph.Eur./USP |
| **Total (film-coated tablet)** | **85.0** | **169.0** | | | |

**TABLE 13P2 QUALITATIVE COMPOSITION OF FILM-COATING MIXTURE**

| **INGREDIENT** | **QUANTITY [% W/W]** | | **FUNCTION** | **REFERENCE TO STANDARDS** |
|---|---|---|---|---|
| | **Film-coating mixture yellow ¹** | **Film-coating mixture yellow ²** | | |
| Hypromellose 2910 ⁴ | 50.000 | 50.000 | Film-forming agent | Ph.Eur./USP |
| Calcium carbonate | 27.369 | 26.950 | Pigment | Ph.Eur./USP |
| Talc | 17.500 | 17.500 | Opacifier | Ph.Eur./USP |
| Macrogols ³ | 5.000 | 5.000 | Film-forming agent | Ph.Eur./NF |
| Iron oxide, yellow | 0.130 | 0.500 | Pigment | E172/NF |
| Iron oxide, red | 0.001 | 0.050 | Pigment | E172/NF |
| **Total (tablet) in mg** | **5.00** | **9.00** | - | - |

| | | | | |
|---|---|---|---|---|
| TABLE KEY: ¹ = See superscript 6 in Table 13P1. This is the film-coating mixture used to coat the 2.5 mg tablet. ² = See superscript 7 in Table 13P1. This is the film-coating mixture used to coat the 5 mg tablet. ³ = polyethylene glycol. ⁴ = hydroxypropyl methylcellulose. | | | | |

## Claims

1. Compound of formula (I) for use in the treatment or prevention of a pathology selected from the group consisting of asthma and chronic obstructive pulmonary disease

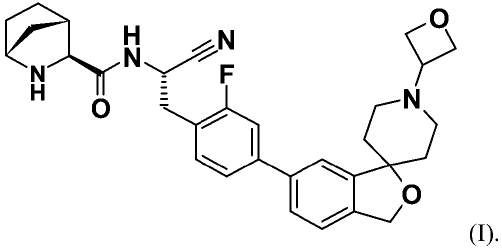

2. Compound of formula (I) for use in the treatment of early disease bronchiectasis, and one or more diseases selected from the group consisting of primary asthma or primary chronic obstructive pulmonary disease (COPD)

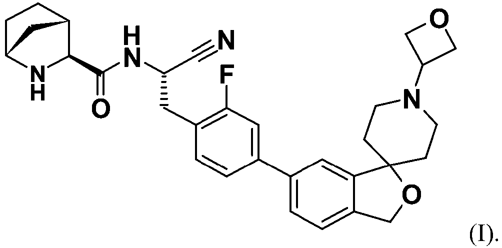

3. Compound (I) for use according to any of the preceding claims, wherein Compound (I) is administered in a daily dose of 1.5 to 5 mg.

4. Compound (I) for use according to any of the preceding claims, wherein Compound (I) is administered in a daily dose of 2.5 mg.

5. Compound (I) for use according to any of the preceding claims, wherein Compound (I) is administered in a daily dose of 5 mg.

6. Compound (I) for use according to any of the preceding claims, wherein Compound (I) is administered orally once daily.

7. Compound (I) for use according to any of the preceding claims, wherein the Compound (I) is the free base, preferably the free base in crystalline anhydrous form.

8. Compound (I) for use according to claim 7, wherein the Compound (I) is in Form [III], having a solid-state NMR spectrum comprising peaks at the following ¹⁹F chemical shifts expressed in parts per million: -112.5 ± 0.2, -116.1 ± 0.2 and -116.5 ± 0.2.

9. Compound (I) for use according to claim 7 or 8, wherein the Compound (I) is in Form [III], having an X-ray powder diffraction pattern comprising peaks at 17.8 ± 0.2, 18.1 ± 0.2, 12.3 ± 0.2, 7.2 ± 0.2 and 6.2 ± 0.2 degrees 2Θ when measured using CuKa radiation.

10. Compound (I) for use according to any of claims 7 to 9, wherein the Compound (I) is in Form [III], having a solid-state NMR spectrum comprising peaks at the following ¹³C chemical shifts expressed in parts per million: -85.2 ± 0.2, -47.3 ± 0.2 and -42.3 ± 0.2.

11. Compound (I) for use according to any of claims 7 to 10, wherein the Compound (I) is in Form [III], having both an X-ray powder diffraction pattern comprising peaks at 17.8 ± 0.2, 18.1 ± 0.2, 12.3 ± 0.2, 7.2 ± 0.2, 6.2 ± 0.2, 18.2 ± 0.2, 17.2 ± 0.2,16.8 ± 0.2, 22.1 ± 0.2 and 19.6 ± 0.2 degrees 2Θ when measured using CuKa radiation and
a solid-state NMR spectrum comprising peaks at the following ¹⁹F chemical shifts expressed in parts per million: -112.5 ± 0.2, -116.1 ± 0.2 and -116.5 ± 0.2.

12. Compound (I) for use according to any of claims 7 to 11, wherein the Compound (I) is in Form [III]obtained by or obtainable by the crystallization of Compound of formula (I) in n-butyl acetate (nBuOAc).

13. A pharmaceutical composition for use in the treatment or prevention of a pathology selected from the group consisting of asthma and chronic obstructive pulmonary disease, wherein the composition comprises the crystalline form according to any of claims 7 to 12 and at least one pharmaceutically acceptable carrier or diluent.

14. The pharmaceutical composition for use according to claim 13, administered in a daily dose of 2.5 mg or 5 mg of crystalline form of the Compound (I).

15. The pharmaceutical composition for use according to any of claims 13 and 14, wherein the pharmaceutical composition is a film-coated tablet having a film coating and a tablet core, wherein the tablet core comprises the crystalline form of the compound of formula (I).
